# EUROPEAN PATENT APPLICATION

(11) **EP 4 406 562 A1**
(43) Date of publication of application: **31.07.2024**
(21) Application number: 21962780.9
(22) Date of filing: 02.11.2021
(51) Int. Cl.: A61L 9/03, B60H 3/00

(54) **FRAGRANCE DISPERSION CONTROL METHOD AND FRAGRANCE DISPERSION APPARATUS**

(71) Applicant: Huawei Technologies Co., Ltd., Shenzhen, Guangdong 518129 (CN)
(72) Inventor: FANG, Shu, Shenzhen, Guangdong 518129 (CN); DONG, Siwei, Shenzhen, Guangdong 518129 (CN); ZHANG, Huimin, Shenzhen, Guangdong 518129 (CN)
(74) Representative: Körber, Martin Hans
(86) International application number: PCT/CN2021/128095
(87) International publication number: WO 2023/077256

(57) **Abstract**

A fragrance dispersing control method and a fragrance dispersing apparatus are provided, which can precisely control fragrance dispersing, avoid excessively fast consumption of perfume, and reduce costs. The fragrance dispersing control method is applied to the fragrance dispersing apparatus, and the fragrance dispersing apparatus may be deployed on a movable device. The method includes obtaining posture information of the fragrance dispersing apparatus, and may include obtaining motion information, a posture angle, and the like of the fragrance dispersing apparatus. In addition, at least one liquid droplet is obtained from a liquid storage tank, and when the fragrance dispersing apparatus is in a moving state, the liquid droplet is controlled, based on the obtained posture information by using the liquid droplet as a unit for an operation, to move along a specified track to a specified area to disperse fragrance.

## Description

### TECHNICAL FIELD

This application relates to the field of air purification, and in particular, to a fragrance dispersing control method and a fragrance dispersing apparatus.

### BACKGROUND

Perfume can relax the body and relax the mind. An apparatus that disperses perfume may be disposed inside a vehicle or indoors, or may be disposed on a movable device, for example, a wearable device, a mobile phone device, a household robot, a household aromatherapy lamp, or a vehicle-mounted device.

However, perfume is a consumable material and is easily consumed. How to avoid excessively fast consumption of the perfume and reduce costs is an urgent problem to be resolved.

### SUMMARY

Embodiments of this application provide a fragrance dispersing control method and a fragrance dispersing apparatus, to precisely control fragrance dispersing, avoid excessively fast consumption of perfume, and reduce costs.

In view of this, a first aspect of this application provides a fragrance dispersing control method. The fragrance dispersing control method is applied to a fragrance dispersing apparatus, and the fragrance dispersing apparatus may be deployed on a movable device, for example, a wearable device, a mobile phone device, a household robot, a household aromatherapy lamp, or a vehicle-mounted device. The method includes obtaining at least one liquid droplet from a liquid storage tank of the fragrance dispersing apparatus. Specifically, the liquid droplet originates directly or indirectly from the liquid storage tank. The liquid storage tank is configured to store at least one type of perfume. Posture information of the fragrance dispersing apparatus is obtained. The posture information is related information used to obtain resistance for a liquid droplet to move along a target track, and may include, for example, motion information of the fragrance dispersing apparatus, and deflection angle information of the fragrance dispersing apparatus in space. The at least one liquid droplet is controlled, based on the posture information of the fragrance dispersing apparatus, to move along a respective target track to a fragrance dispersing area.

In the solution provided in the first aspect, a trace amount of liquid, for example, one liquid droplet, is obtained from the liquid storage tank of the fragrance dispersing apparatus, and fragrance dispersing can be precisely controlled by using the liquid droplet as a unit during a fragrance dispersing process. In addition, the fragrance dispersing apparatus may be deployed on the movable device. In some scenarios, the device cannot always remain stationary, and even moves frequently. Movement of the liquid droplet can be precisely controlled to ensure precise control of fragrance dispersing under these scenarios. Posture information of the device may also be obtained, and movement resistance for the liquid droplet caused by movement of the device is estimated based on the posture information of the device, thereby eliminating the movement resistance that affects the liquid droplet to move along a planned track. In this way, even if the fragrance dispersing apparatus is deployed on the movable device, the liquid droplet can be precisely controlled to achieve precise control of fragrance dispersing.

In a possible implementation of the first aspect, the method further includes heating the liquid droplet in the fragrance dispersing area. In this implementation, heating the at least one liquid droplet in the fragrance dispersing area can speed up fragrance dispersing.

In a possible implementation of the first aspect, that the at least one liquid droplet is controlled, based on the posture information of the fragrance dispersing apparatus, to move along a respective target track to a fragrance dispersing area includes: The at least one liquid droplet is controlled, based on the posture information of the fragrance dispersing apparatus by using a first microfluidic circuit, to move along the respective target track to the fragrance dispersing area. In this implementation, the liquid droplet can be controlled to move along the target track by using the microfluidic circuit. This increases diversity of the solution.

In a possible implementation of the first aspect, that the at least one liquid droplet is controlled, based on the posture information of the fragrance dispersing apparatus by using a first microfluidic circuit, to move along the respective target track to the fragrance dispersing area includes: Resistance for the at least one liquid droplet to move along a respective target track is obtained based on the current posture information of the fragrance dispersing apparatus; and the at least one liquid droplet is driven, based on the resistance and a first driving force by using the first microfluidic circuit, to move along the respective target track to the fragrance dispersing area. The first driving force includes a driving force that drives the at least one liquid droplet to move along the respective target track on a horizontal plane. In this implementation, whether the device flips, vibrates, shakes, or the like is detected. If it is detected that the device is in a moving state, the posture information of the device is obtained. A control force applied to the liquid droplet is adjusted based on the posture information of the device, to ensure that the liquid droplet can also move normally on the target track even if the fragrance dispersing apparatus is in the moving state, and ensure precise control of fragrance dispersing.

In a possible implementation of the first aspect, the first driving force indicates to apply a first voltage value at a first target position, and that the at least one liquid droplet is driven, based on the resistance and a first driving force by using the first microfluidic circuit, to move along the respective target track to the fragrance dispersing area includes: A second driving force is obtained based on the resistance and a mapping relationship, where the second driving force indicates to apply a second voltage value at a second target position, and the mapping relationship includes a mapping between the resistance, the second target position, and the second voltage value; and the first voltage value is applied at the first target position and the second voltage value is applied at the second target position simultaneously by using the first microfluidic circuit, to drive the at least one liquid droplet to move along the respective target track to the fragrance dispersing area. In this implementation, whether the device flips, vibrates, shakes, or the like is detected. If it is detected that the device is in the moving state, the posture information of the device is obtained. A voltage applied to an electrode in the microfluidic circuit is adjusted based on the posture information of the device, to ensure that the liquid droplet can move normally on the target track even if the fragrance dispersing apparatus is in the moving state, and ensure precise control of fragrance dispersing.

In a possible implementation of the first aspect, the obtaining at least one liquid droplet from a liquid storage tank of the fragrance dispersing apparatus includes: obtaining a liquid droplet from the liquid storage tank of the fragrance dispersing apparatus by using a first extraction apparatus. The first extraction apparatus includes at least one of a capillary tube, a liquid pump, a microfluidic circuit or a combination apparatus. The combination apparatus includes a valve and the microfluidic circuit. One end of the valve is connected to the liquid storage tank, and the other end of the valve is connected to the microfluidic circuit. When the valve is open, the liquid droplet is obtained from the liquid storage tank by using the microfluidic circuit. In this implementation, a manner for obtaining the liquid droplet may be selected based on a size of the fragrance dispersing apparatus or installation space of the fragrance dispersing apparatus. For example, if the fragrance dispersing apparatus is deployed on a large device, or the installation space of the fragrance dispersing apparatus is large, candidate liquid may be obtained from the liquid storage tank to a liquid supply area by using the liquid pump. In this implementation, the installation space of the fragrance dispersing apparatus is large. In these scenarios, a large area of fragrance dispersing is usually required. Therefore, a large amount of liquid may need to be obtained from the liquid storage tank to the liquid supply area. Therefore, a manner of using the liquid pump is more appropriate. If the fragrance dispersing apparatus is deployed on a small device, or the installation space of the fragrance dispersing apparatus is small, the candidate liquid may be obtained from the liquid storage tank to the liquid supply area by using the capillary tube or the combination apparatus. In this implementation, the installation space of the fragrance dispersing apparatus is small. In these scenarios, a small area of fragrance dispersing is usually required. Therefore, a small amount of liquid may need to be obtained from the liquid storage tank to the liquid supply area. Therefore, a manner of using the capillary tube or the combination apparatus is more appropriate.

In a possible implementation of the first aspect, the obtaining at least one liquid droplet from a liquid storage tank of the fragrance dispersing apparatus includes: obtaining the at least one liquid droplet from the liquid storage tank of the fragrance dispersing apparatus by using a second microfluidic circuit.

In a possible implementation of the first aspect, the obtaining the at least one liquid droplet from the liquid storage tank of the fragrance dispersing apparatus by using a second microfluidic circuit includes: obtaining the candidate liquid from the liquid storage tank of the fragrance dispersing apparatus; and splitting the liquid by using the second microfluidic circuit, to obtain the liquid droplet.

In a possible implementation of the first aspect, the obtaining the liquid droplet from a liquid storage tank of the fragrance dispersing apparatus includes: obtaining a plurality of liquid droplets from different liquid storage tanks of the fragrance dispersing apparatus. That the at least one liquid droplet is controlled, based on the posture information of the fragrance dispersing apparatus, to move along a respective target track to a fragrance dispersing area includes: controlling, based on the posture information of the fragrance dispersing apparatus, at least two of the plurality of liquid droplets to move along respective target tracks to obtain a mixed liquid droplet. The mixed liquid droplet is controlled, based on the posture information of the fragrance dispersing apparatus, to move along a target track of the mixed liquid droplet to the fragrance dispersing area. In this implementation, the fragrance dispersing apparatus supports a fragrance mixing mode to enhance user experience.

In a possible implementation of the first aspect, the obtaining at least one liquid droplet from a liquid storage tank of the fragrance dispersing apparatus includes: obtaining a plurality of liquid droplets from different liquid storage tanks of the fragrance dispersing apparatus; and that the at least one liquid droplet is controlled, based on the posture information of the fragrance dispersing apparatus, to move along a respective target track to a fragrance dispersing area includes: controlling, based on the posture information of the fragrance dispersing apparatus, at least two of the plurality of liquid droplets to move along respective target tracks to a same position in the fragrance dispersing area.

In a possible implementation of the first aspect, the method further includes: obtaining a first instruction, where the first instruction indicates at least one of a fragrance type, fragrance concentration, or fragrance dispersing duration. The obtaining the liquid droplet from a liquid storage tank of the fragrance dispersing apparatus includes: obtaining at least one liquid droplet from at least one liquid storage tank of the fragrance dispersing apparatus in response to the first instruction. In this implementation, the user may interact with the fragrance dispersing apparatus, so that the fragrance dispersing apparatus performs fragrance dispersing based on a requirement of the user, thereby improving user experience.

In a possible implementation of the first aspect, the method further includes: obtaining a second instruction, where the second instruction indicates at least one target track. That the at least one liquid droplet is controlled, based on the posture information of the fragrance dispersing apparatus, to move along a respective target track to a fragrance dispersing area includes: controlling, based on the posture information of the fragrance dispersing apparatus in response to the second instruction, the at least one liquid droplet to move along the at least one target track to the fragrance dispersing area. In this implementation, the user may interact with the fragrance dispersing apparatus, so that a target track is planned according to an instruction of the user in the fragrance dispersing apparatus, thereby increasing interest of the solution and improving user experience.

In a possible implementation of the first aspect, the fragrance dispersing apparatus is deployed on at least one of a mobile phone case, a mobile phone rear cover, a subscriber identity module SIM card tray, an electronic device, an in-vehicle apparatus, or a robot.

In a possible implementation of the first aspect, an inertial measurement unit IMU is deployed on the fragrance dispersing apparatus, and the obtaining posture information of the fragrance dispersing apparatus includes: obtaining the posture information of the fragrance dispersing apparatus based on IMU data.

A second aspect of this application provides a fragrance dispersing apparatus. The fragrance dispersing apparatus includes a liquid storage tank, a processor, a liquid droplet splitting apparatus, and a liquid droplet movement platform. The liquid droplet splitting apparatus is configured to obtain at least one liquid droplet from the liquid storage tank of the fragrance dispersing apparatus. The processor is configured to control, based on posture information of the fragrance dispersing apparatus, the at least one liquid droplet to move from the liquid droplet movement platform along a target track to a fragrance dispersing area.

In a possible implementation of the second aspect, the fragrance dispersing apparatus further includes a heating apparatus. The heating apparatus is configured to heat the at least one liquid droplet in the fragrance dispersing area.

In a possible implementation of the second aspect, the processor is specifically configured to control, based on the posture information of the fragrance dispersing apparatus by using a first microfluidic circuit, the at least one liquid droplet to move along the respective target track to the fragrance dispersing area.

In a possible implementation of the second aspect, the processor is specifically configured to: obtain, based on the current posture information of the fragrance dispersing apparatus, resistance for the at least one liquid droplet to move along the respective target track; and drive, based on the resistance and a first driving force by using a first microfluidic circuit, the at least one liquid droplet to move along the respective target track to the fragrance dispersing area. The first driving force includes a driving force that drives the at least one liquid droplet to move along the respective target track on a horizontal plane.

In a possible implementation of the second aspect, the first driving force indicates to apply a first voltage value at a first target position. The processor is specifically configured to: obtain a second driving force based on the resistance and a mapping relationship, where the second driving force indicates to apply a second voltage value at a second target position, and the mapping relationship includes a mapping between the resistance, the second target position, and the second voltage value; and simultaneously apply the first voltage value at the first target position and apply the second voltage value at the second target position by using the first microfluidic circuit, to drive the at least one liquid droplet to move along the respective target track to the fragrance dispersing area.

In a possible implementation of the second aspect, the liquid droplet splitting apparatus is specifically configured to obtain a liquid droplet from the liquid storage tank of the fragrance dispersing apparatus by using a capillary tube or a liquid pump.

In a possible implementation of the second aspect, the liquid droplet splitting apparatus is specifically configured to obtain a liquid droplet from the liquid storage tank of the fragrance dispersing apparatus by using a second microfluidic circuit.

In a possible implementation of the second aspect, the liquid droplet splitting apparatus is specifically configured to: obtain candidate liquid from the liquid storage tank of the fragrance dispersing apparatus; and split the candidate liquid by using the second microfluidic circuit, to obtain a liquid droplet.

In a possible implementation of the second aspect, the liquid droplet splitting apparatus is specifically configured to obtain a plurality of liquid droplets from different liquid storage tanks of the fragrance dispersing apparatus. The processor is specifically configured to: control, based on the posture information of the fragrance dispersing apparatus, at least two of the plurality of liquid droplets to move to a same position along respective target tracks, to obtain a mixed liquid droplet; and control, based on the posture information of the fragrance dispersing apparatus, the mixed liquid droplet to move along a target track of the mixed liquid droplet to the fragrance dispersing area.

In a possible implementation of the second aspect, the liquid droplet splitting apparatus is specifically configured to obtain a plurality of liquid droplets from different liquid storage tanks of the fragrance dispersing apparatus. The processor is specifically configured to control, based on the posture information of the fragrance dispersing apparatus, at least two of the plurality of liquid droplets to move along respective target tracks to a same position in the fragrance dispersing area.

In a possible implementation of the second aspect, the liquid droplet splitting apparatus is specifically configured to obtain at least one liquid droplet from at least one liquid storage tank of the fragrance dispersing apparatus in response to a first instruction. The first instruction indicates at least one of a fragrance type, fragrance concentration, or fragrance dispersing duration.

In a possible implementation of the second aspect, the processor is specifically configured to control, based on the posture information of the fragrance dispersing apparatus in response to a second instruction, at least one liquid droplet to move along at least one target track to the fragrance dispersing area. The second instruction indicates the at least one target track.

In a possible implementation of the second aspect, the apparatus further includes a housing. The liquid droplet movement platform is accommodated in a housing, and transparency of the housing is greater than a threshold.

In a possible implementation of the second aspect, the fragrance dispersing apparatus is deployed on at least one of a mobile phone case, a mobile phone rear cover, a subscriber identity module SIM card tray, an electronic device, an in-vehicle apparatus, or a robot.

In a possible implementation of the second aspect, the fragrance dispersing apparatus further includes an inertial measurement unit IMU. The IMU is configured to obtain the posture information of the fragrance dispersing apparatus.

A third aspect of this application provides a fragrance dispersing apparatus, including a memory, configured to store computer-readable instructions. The fragrance dispersing apparatus may further include a processor coupled to the memory, and the processor is configured to execute the computer-readable instructions in the memory to perform the method described in any one of the first aspect or the possible implementations of the first aspect.

A fourth aspect of this application provides a computer-readable storage medium, including instructions. When the instructions are run on a computer apparatus, the computer apparatus is enabled to perform the method described in any one of the first aspect or the possible implementations of the first aspect.

A fifth aspect of this application provides a chip. The chip is coupled with a memory and is configured to execute a program stored in the memory, to perform the method described in any one of the first aspect or the possible implementations of the first aspect.

A sixth aspect of this application provides a computer program product. When the computer program product runs on one or more processors, the method described in any one of the first aspect or the possible implementations of the first aspect is performed.

For beneficial effect brought by the second aspect to the sixth aspect, refer to the beneficial effect brought by the first aspect and the implementations of the first aspect for understanding. Details are not described herein again.

### BRIEF DESCRIPTION OF DRAWINGS

FIG. 1a is a schematic diagram of an application scenario of a fragrance dispersing apparatus according to an embodiment of this application;
FIG. 1b is a schematic diagram of an application scenario of another fragrance dispersing apparatus according to an embodiment of this application;
FIG. 1c is a schematic diagram of an application scenario of another fragrance dispersing apparatus according to an embodiment of this application;
FIG. 1d is a schematic diagram of an application scenario of another fragrance dispersing apparatus according to an embodiment of this application;
FIG. 2a is a schematic diagram of a structure of a fragrance dispersing apparatus according to an embodiment of this application;
FIG. 2b is a schematic diagram of a principle of droplet splitting;
FIG. 3 is a schematic diagram of a structure of another fragrance dispersing apparatus according to an embodiment of this application;
FIG. 4a is a schematic diagram of a structure of another fragrance dispersing apparatus according to an embodiment of this application;
FIG. 4b is a schematic diagram of a structure of another fragrance dispersing apparatus according to an embodiment of this application;
FIG. 5 is a schematic diagram of a working mode of a piezoelectric driven liquid pump;
FIG. 6 is a schematic diagram of an application scenario of another fragrance dispersing apparatus according to an embodiment of this application;
FIG. 7 is a schematic diagram of adjusting a voltage applied to an electrode based on posture information;
FIG. 8a is a schematic diagram of a structure of another fragrance dispersing apparatus according to an embodiment of this application;
FIG. 8b is a schematic diagram of a structure of another fragrance dispersing apparatus according to an embodiment of this application;
FIG. 8c is a schematic diagram of a structure of another fragrance dispersing apparatus according to an embodiment of this application;
FIG. 9a is a schematic diagram of an application scenario of another fragrance dispersing apparatus according to an embodiment of this application;
FIG. 9b is a schematic diagram of an application scenario of another fragrance dispersing apparatus according to an embodiment of this application;
FIG. 10 is a schematic flowchart of a fragrance dispersing control method according to an embodiment of this application;
FIG. 11 is a schematic flowchart of another fragrance dispersing control method according to an embodiment of this application;
FIG. 12 is a schematic flowchart of a fragrance dispersing control method according to an embodiment of this application;
FIG. 13 is a schematic flowchart of another fragrance dispersing control method according to an embodiment of this application;
FIG. 14 is a schematic diagram of a structure of a fragrance dispersing apparatus according to an embodiment of this application; and
FIG. 15 is a schematic diagram of a structure of an electronic device according to an embodiment of this application.

### DESCRIPTION OF EMBODIMENTS

The following describes technical solutions in embodiments of this application with reference to accompanying drawings in embodiments of this application. It is clear that the described embodiments are merely some rather than all of embodiments of this application. All other embodiments obtained by a person skilled in the art based on embodiments of this application without creative efforts shall fall within the protection scope of this application.

A fragrance dispersing apparatus described in embodiments of this application is an apparatus that uses volatilization of odorous liquids such as perfume and essential oils to achieve fragrance dispersing. A use manner includes heating, natural placement, or the like. In this application, the fragrance dispersing apparatus is also sometimes referred to as a fragrance/aromatherapy/perfume dispersing apparatus, fragrance, aromatherapy, or the like. It should be understood that they express a same meaning when their difference is not specifically emphasized. In addition, it should be noted that, to simplify descriptions, odorous liquid in the fragrance dispersing apparatus is collectively referred to as perfume in the following embodiments.

Because the perfume is easily consumed, a user needs to replace the perfume frequently. This is not conducive to reducing costs of the fragrance dispersing apparatus. Moreover, with increasingly extensive application of the fragrance dispersing apparatus, improving user experience of using the fragrance dispersing apparatus has become a research trend.

To resolve this problem, embodiments of this application provide a fragrance dispersing control method and a fragrance dispersing apparatus. Fragrance dispersing is precisely controlled, to reduce consumption of perfume. In addition, different fragrance dispersing manners may be provided based on a requirement of the user, to improve user experience of using the fragrance dispersing apparatus.

To better understand the solutions provided in embodiments of this application, the following first describes a research idea of the solutions provided in embodiments of this application.

Digital microfluidics (digital microfluidics, DMF), also referred to as microfluidics, can use a liquid droplet in a microliter to nanoliter range for precise manipulation to implement complex laboratory analysis. Common applications include polymerase chain reaction (polymerase chain reaction, PCR), deoxyribonucleic acid (deoxyribonucleic acid, DNA) sequencing, protein analysis and other applications in biological, medical, and chemical fields.

At present, a digital microfluidic technology has not been applied to the fragrance dispersing apparatus to precisely control fragrance dispersing. In embodiments of this application, it is first thought that the digital microfluidic technology or another technology that can precisely operate a liquid droplet may be used, to obtain a liquid droplet from a perfume pool of the fragrance dispersing apparatus, and operate the liquid droplet to precisely control fragrance dispersing.

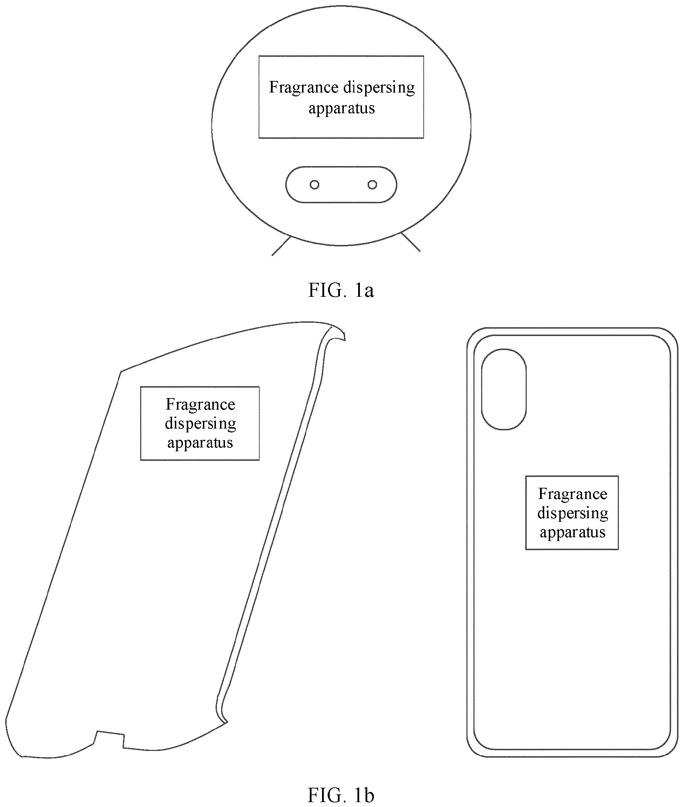

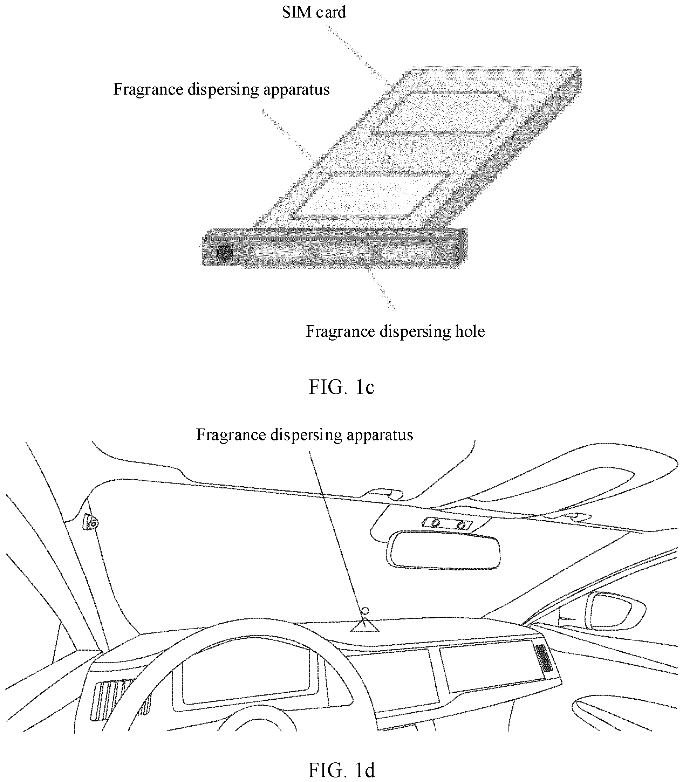

In addition, the applicant further considers that only the operation on the liquid droplet cannot meet requirements of precise control of fragrance dispersing in all application scenarios. Specifically, the microfluidic technology is usually used in biomedical test scenarios. In these scenarios, an operation platform on which the microfluidic technology is applied is stable and stationary, so that microfluidic effect can be achieved, that is, precise control of the liquid droplet can be achieved. However, the fragrance dispersing apparatus may be deployed on a movable device, for example, a wearable device, a mobile phone device, a household robot, a household aromatherapy lamp, or a vehicle-mounted device. In these scenarios, the device cannot always remain stationary, and even moves frequently. Consequently, precise control of fragrance dispersing cannot be achieved even the microfluidic technology is applied directly to the fragrance dispersing apparatuses on these movable devices. For example, FIG. 1a to FIG. 1d are schematic diagrams of structures in which a fragrance dispersing apparatus is deployed on a movable device. As shown in FIG. 1a, the fragrance dispersing apparatus may be deployed on a floor sweeping robot. As shown in FIG. 1b, the fragrance dispersing apparatus may be deployed on a housing of a mobile phone or a mobile phone case. As shown in FIG. 1c, the fragrance dispersing apparatus may be deployed on a SIM card. As shown in FIG. 1d, the fragrance dispersing apparatus may be deployed on a vehicle-mounted device.

Therefore, based on the foregoing consideration, the applicant further considers that posture information of the device may be obtained, and resistance for the liquid droplet caused by movement of the device may be estimated based on the posture information of the device, thereby eliminating resistance that affects movement of the liquid droplet based on a planned track. In this way, even if the fragrance dispersing apparatus is deployed on the movable device, the liquid droplet can be precisely controlled to achieve precise control of fragrance dispersing.

Because an operation needs to be performed on a liquid droplet in this embodiment of this application, it should be noted that any technology in which a precise operation can be performed on the liquid droplet may be used in this embodiment of this application. For example, the following uses the digital microfluidic technology as an example to describe how to perform a precise operation on the liquid droplet.

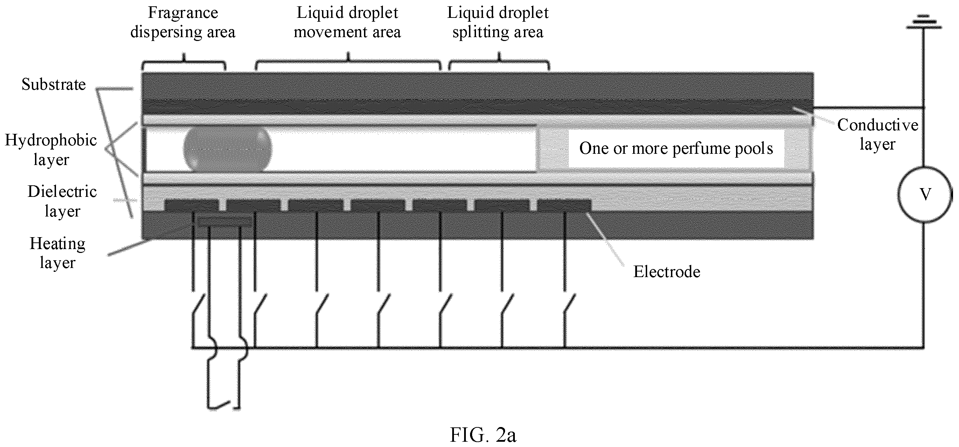

A basic working principle of the digital microfluidic technology is as follows: The digital microfluidic technology relies on liquid droplet formation caused by liquid surface tension. Polar hydrophilicity of a liquid surface is created by using an electric field, to flatten the liquid droplet. A polarization position is controlled to generate a tension gradient, so that a controlled droplet is displaced on a microfluidic platform. The digital microfluidic platform is disposed based on a substrate and an electrode, and precise displacement of the liquid droplet is controlled through voltage. Electrodes are patterned in an array on a bottom layer, and continuous electrodes are located on a top layer. When a voltage is applied, the electrode is activated and the liquid droplet can be manipulated by a linear array potential change along an electrode line. To better understand the digital microfluidic technology, the following uses an example with reference to FIG. 2a.

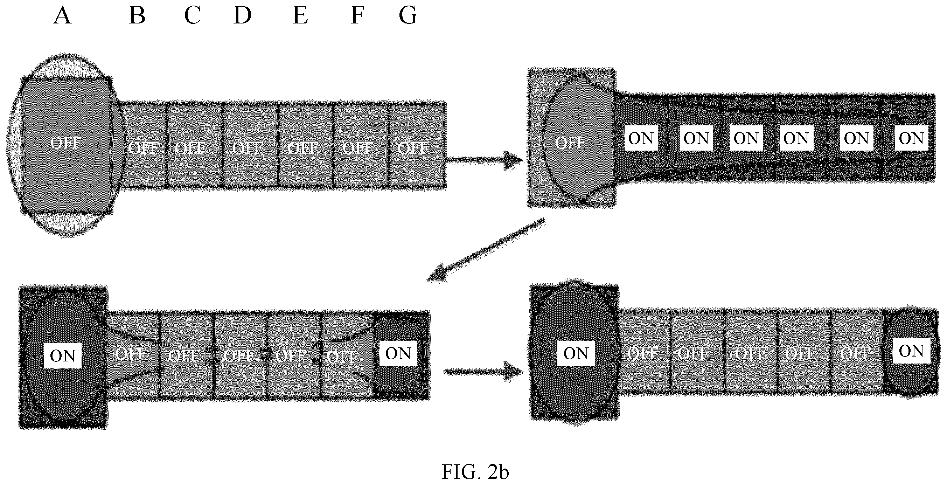

FIG. 2a is a schematic diagram of a structure of a microfluidic circuit according to an embodiment of this application. The microfluidic circuit may include a substrate, a hydrophobic layer, a dielectric layer, a perfume pool, a liquid droplet splitting area, a liquid droplet movement area, and a fragrance dispersing area. The substrate may be a glass substrate, or may be an ITO glass conductive layer. This is not limited in embodiments of this application. A main component of the ITO is Indium tin oxideindium tin oxide. The dielectric layer, the conductive layer and the electrode constitute a control circuit, and the control circuit is located between two substrates. A lower layer is a drive electrode array, and the dielectric layer may be prepared on the drive electrode array. The conductive layer is disposed in an upper layer. A voltage is supplied to a corresponding position by controlling a switch corresponding to the electrode array, to achieve liquid droplet splitting and allow the liquid droplet to move according to the track in the liquid droplet movement area. The hydrophobic layer may be Teflon or another chemical or physical hydrophobic material, and the layer is a movement channel for a micro droplet. The dielectric layer may be a material, for example, silicon dioxide or parylene. This is not limited in embodiments of this application. A liquid storage tank includes one or more independent perfume pools containing perfume liquid, and a perfume liquid droplet may be extracted by using a switch, a pump, a capillary tube, or the like. The liquid storage tank is described in detail below. The liquid droplet splitting area is close to the perfume pool, and when the perfume liquid is extracted from each perfume pool independently by using the pump, the capillary tube, or through voltage drive, the liquid droplet is split in this area. A split liquid droplet can move to the fragrance dispersing area under an action of the control circuit, and a heating layer may be disposed in the fragrance dispersing area to heat the liquid droplet to achieve fragrance dispersing. With reference to FIG. 2b, a process of splitting the liquid droplet is specifically described below.

Assuming that a position of a candidate liquid droplet is A, voltages of consecutive adjacent electrodes (B, C, D, E, F, G) in a specific direction where the candidate liquid droplet is located are turned on, to lengthen the candidate liquid droplet in this direction. A voltage of the initial position A of the candidate liquid droplet is turned on, voltages of the consecutive adjacent electrodes (B, C, D, E, F) are turned off, and a voltage of the electrode G furthest from the initial position A of the candidate liquid droplet is maintained. Under pulling action of both ends, the liquid droplet is split to complete the process of splitting the liquid droplet.

The following describes the solutions provided in embodiments of this application based on the foregoing research ideas.

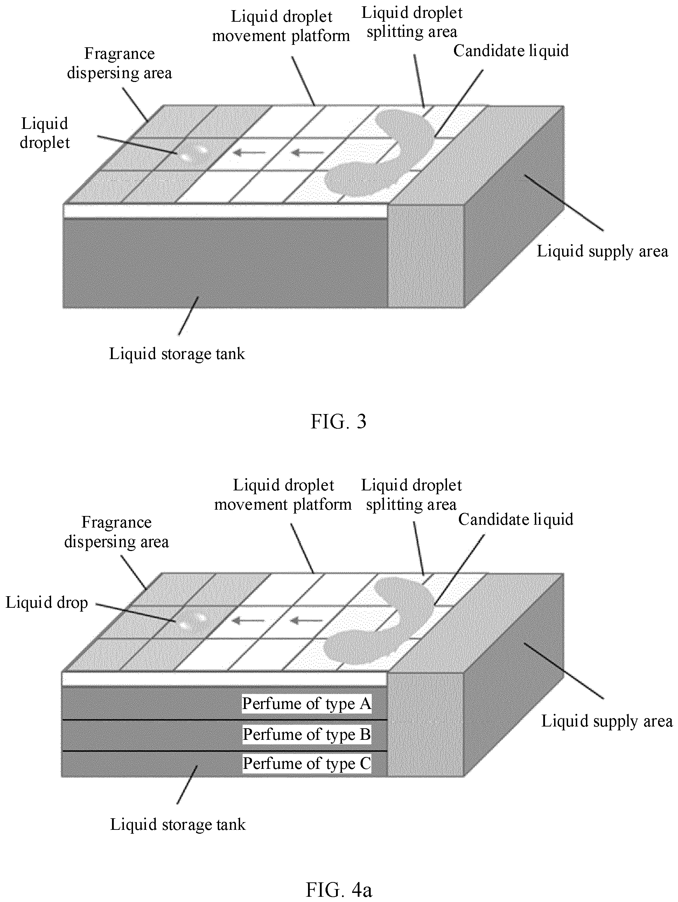

FIG. 3 is a schematic diagram of a structure of a fragrance dispersing apparatus according to an embodiment of this application.

As shown in FIG. 3, the fragrance dispersing apparatus provided in this embodiment of this application includes a liquid storage tank, a liquid supply area, a liquid droplet splitting area, a liquid droplet movement platform, and a fragrance dispersing area. The following describes these structures respectively.

### 1. Liquid storage tank

The liquid storage tank is used to store perfume.

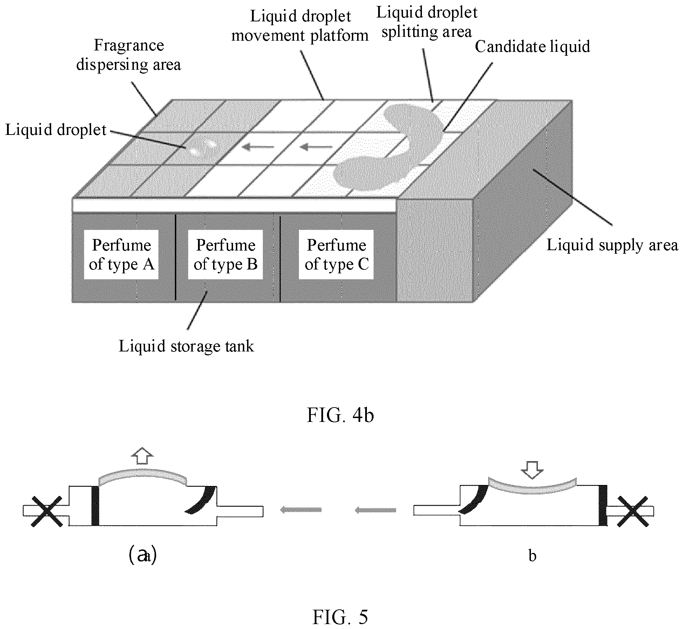

In a possible implementation, the liquid storage tank may include one or more areas for storing perfume, and each area may be used to store one type of perfume. If the liquid storage tank includes a plurality of areas for storing perfume, the plurality of areas may be arranged in a plurality of manners. This is not limited in this embodiment of this application. Refer to FIG. 4a and FIG. 4b. Two possible arrangement manners are provided. As shown in FIG. 4a, areas may be arranged in a superimposed manner. As shown in FIG. 4b, areas may alternatively be arranged side by side. In embodiments of this application, sometimes, each of the plurality of areas for storing perfume is alternatively referred to as one liquid storage tank, that is, it may be considered that the fragrance dispersing apparatus includes a plurality of liquid storage tanks. Actually, this expression is essentially the same as that one liquid storage tank includes one or more areas. Both expressions indicate that one or more types of perfume may be stored in the solution provided in embodiments of this application.

In a possible implementation, at least one of the one or more areas for storing perfume is detachable. In this implementation, a user is allowed to freely replace the one or more storage areas, or the user may remove the one or more storage areas from the storage pool to facilitate addition of perfume.

In a possible implementation, each type of perfume may have a unique number identifier, so that the fragrance dispersing apparatus obtains, based on the number identifier indicated in an obtained instruction, a type of perfume corresponding to the number identifier.

### 2. Liquid supply area

The liquid supply area is used to obtain candidate liquid from the liquid storage tank.

In a possible implementation, the liquid supply area obtains a specific amount of liquid from the liquid storage tank at intervals of preset duration.

In a possible implementation, when an amount of the obtained candidate liquid in the liquid supply area is less than a preset threshold, a specific amount of liquid is obtained from the liquid storage tank, so that an amount of the candidate liquid in the liquid supply area reaches the preset threshold.

The liquid supply area may obtain the candidate liquid from the liquid storage tank in a plurality of manners, including but not limited to at least one of a manner of using a capillary tube, a manner of using a liquid pump, or a manner of using a microfluidic circuit. A manner of obtaining the candidate liquid from the liquid storage tank may be selected based on a size of a device deployed with the fragrance dispersing apparatus or a size of installation space of the fragrance dispersing apparatus. The following describes this by using examples with reference to several implementations.

In a possible implementation, if the fragrance dispersing apparatus is deployed on a large device, or installation space of the fragrance dispersing apparatus is large, the candidate liquid may be obtained from the liquid storage tank to the liquid supply area by using the liquid pump. In this implementation, the installation space of the fragrance dispersing apparatus is large. In these scenarios, a large area of fragrance dispersing is usually required. Therefore, a large amount of liquid may need to be obtained from the liquid storage tank to the liquid supply area. Therefore, the manner of using the liquid pump is more appropriate. For example, this manner is suitable when the fragrance dispersing apparatus is deployed on a mobile robot, for example, a floor sweeping robot or a carrier robot.

A driving mode of the liquid pump is not limited in embodiments of this application. For example, any driving mode, for example, piezoelectric drive, electrostatic drive, electromagnetic drive, drive by using a memory alloy, and drive by using electro-hydraulic power may be used. The following uses piezoelectric drive as an example to describe a working mode of the liquid pump. FIG. 5 is a schematic diagram of a working mode of a piezoelectric driven liquid pump. a in FIG. 5 and b in FIG. 5 are two different states of the liquid pump. The liquid pump includes a diaphragm, a microflow channel, two one-way valves, and another structure. A right valve is connected to the liquid storage tank, and a left valve is connected to the liquid droplet splitting area. As shown in a in FIG. 5, the diaphragm vibrates upwards through piezoelectric drive, the left valve is closed, and the right valve is opened to absorb water from the right side. As shown in b in FIG. 5, the diaphragm vibrates downwards through piezoelectric drive, the left valve is opened and the right valve is closed, and water is drained from the left valve.

In a possible implementation, if the fragrance dispersing apparatus is deployed on a small device, or installation space of the fragrance dispersing apparatus is small, the candidate liquid may be obtained from the liquid storage tank to the liquid supply area by using the capillary tube. In this implementation, the installation space of the fragrance dispersing apparatus is small. In these scenarios, a small area of fragrance dispersing is usually required. Therefore, a small amount of liquid may need to be obtained from the liquid storage tank to the liquid supply area. Therefore, the manner of using the capillary tube is more appropriate. A working principle of the capillary tube is to utilize a capillary phenomenon. The capillary phenomenon means that a joint action of surface tension, cohesion and adhesion of liquid allows water to rise to a specific height in the capillary tube with a small diameter.

For example, the fragrance dispersing apparatus is deployed on a mobile phone or a subscriber identity module (subscriber identity module, SIM) card.

A capillary tube end may be disposed in the liquid droplet splitting area. After a liquid droplet is split from the capillary tube end on the liquid splitting area through the microfluidic technology, for example, a liquid droplet of 0.5 µL is split from the capillary tube end. In this case, the capillary tube automatically absorbs a same amount of perfume from the liquid storage tank under an action of suction of the capillary tube. For example, a liquid droplet of 0.5 µL is automatically sucked from the liquid storage tank to fill the split liquid droplet of 0.5 µL.

In a possible implementation, if the fragrance dispersing apparatus is deployed on a small device, or installation space of the fragrance dispersing apparatus is small, the candidate liquid may be obtained from the liquid storage tank to the liquid supply area by using a valve and a microfluidic circuit. In this implementation, a specific amount of liquid may be obtained from the liquid storage tank to the candidate liquid supply area by using the microfluidic circuit. One end of the valve is connected to the liquid storage tank, and the other end of the valve is connected to the microfluidic circuit. When the valve is open, the liquid droplet is obtained from the liquid storage tank by using the microfluidic circuit.

In a possible implementation, liquid in the liquid supply area may alternatively be returned to the liquid storage tank. For example, in some scenarios, according to one fragrance dispersing instruction, perfume of type A needs to be obtained to the liquid supply area. If another fragrance dispersing instruction is converted, perfume of type B needs to be obtained to the liquid supply area. Remaining perfume of type A in the liquid supply area may be first returned to the liquid storage tank, and then the perfume of type B may be re-obtained from the liquid storage tank to the liquid supply area.

In addition, in a possible implementation, a plurality of liquid supply areas may be included, and one liquid supply area is separately disposed for each type of perfume. For example, the perfume of type A is extracted to only a liquid supply area corresponding to the perfume of type A, and the perfume of type B is extracted to only a liquid supply area corresponding to the perfume of type B, to ensure that perfume in the liquid supply area is not contaminated by another type of perfume.

It should be noted that, in some implementations, the liquid supply area is an optional structure, in other words, the liquid supply area may not be disposed in the fragrance dispersing apparatus.

### 3. Liquid droplet splitting area

The liquid droplet splitting area is used to split liquid in the liquid supply area to obtain a liquid droplet.

How to split the liquid has been described in the foregoing descriptions of digital microfluidics. Details are not described herein again. It should be noted that, in this embodiment of this application, the liquid may alternatively be split by using a liquid splitting manner other than the digital microfluidic technology, to obtain the liquid droplet.

In a possible implementation, when an amount of the liquid in the liquid supply area obtained by the liquid droplet splitting area is less than a threshold, and when a liquid droplet splitting operation is affected, a notification may be sent to the liquid supply area, so that the liquid supply area that receives the notification obtains the candidate liquid from the liquid storage tank. In a possible implementation, when the amount of the liquid in the liquid supply area obtained by the liquid droplet splitting area is less than the threshold, and when a liquid droplet splitting operation is affected, liquid may alternatively be directly obtained from the liquid storage tank, and the liquid in the liquid storage tank is split.

As described above, in some implementations, the liquid supply area is an optional structure. In these embodiments, the liquid droplet splitting area may directly split the liquid in the liquid storage tank to obtain the liquid droplet.

### 4. Liquid droplet movement platform

The liquid droplet movement platform is used to enable a liquid droplet to move along a target track to the fragrance dispersing area. In embodiments of this application, the target track may also be referred to as a planned track or a planned moving track, and the target track, the planned track and the planned moving track have the same meaning.

First, based on the solutions provided in embodiments of this application, the target track may be obtained in different manners. In a possible implementation, at least one target track may be specified in advance. In a possible implementation, at least one target track may be obtained according to an instruction of the user. The following separately describes the two implementations by using examples.

In a possible implementation, at least one target track may be specified in advance. For example, if there is only one type of perfume in the liquid storage tank, after the liquid droplet splitting area obtains a liquid droplet, a moving track of the liquid droplet on the liquid droplet movement platform may be specified in advance. For another example, there are a plurality of types of perfume in the liquid storage tank, after the liquid droplet splitting area obtains liquid droplets of different types of perfume, a moving track of a liquid droplet of each type of perfume on the liquid droplet movement platform may be specified in advance. For another example, in some implementations, if liquid droplets of a plurality of different types of perfume need to be mixed to obtain mixed fragrance, moving tracks of liquid droplets of to-be-mixed perfume may be specified in advance, so that liquid droplets of the to-be-mixed perfume move to a specified area along pre-specified tracks, to complete mixing. In a possible implementation, a shortest path line between the liquid droplet splitting area and the fragrance dispersing area may be specified as the target track in advance.

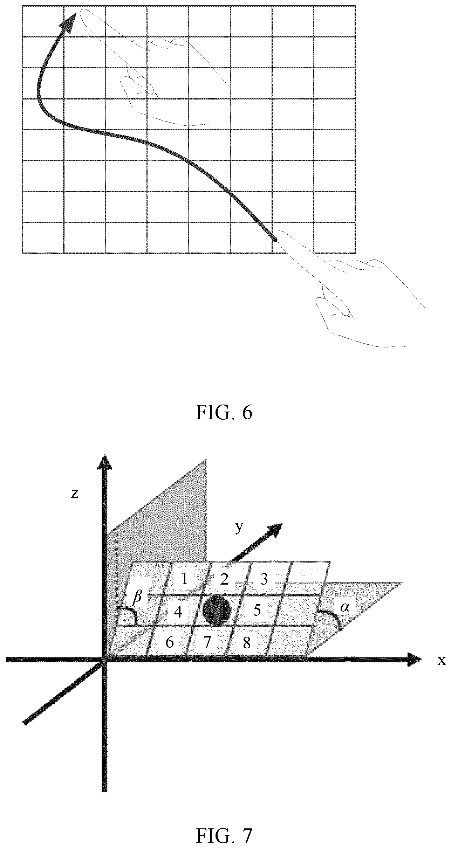

In a possible implementation, at least one target track may alternatively be obtained according to an instruction of the user. Refer to FIG. 6. In this implementation, the user may interact with the fragrance dispersing apparatus to send an instruction to the fragrance dispersing apparatus to indicate the target track. For example, a touch area is disposed in the fragrance dispersing apparatus. The user may draw a track in the touch area. After obtaining the drawn track, the fragrance dispersing apparatus determines that the drawn track is the target track.

Then, based on the solutions provided in embodiments of this application, a plurality of liquid droplets may be simultaneously controlled to move to the fragrance dispersing area along their respective target tracks. In a possible implementation, for liquid droplets from a same area for storing perfume, target tracks of the liquid droplets may be the same or different. In other words, for liquid droplets of a same type of perfume, these liquid droplets may move along a same target track or may move along different target tracks. For liquid droplets from different areas for storing perfume, target tracks of the liquid droplets may be the same or different if the liquid droplets do not move synchronously. If the liquid droplets from different areas for storing perfume move synchronously, the target tracks of the liquid droplets are different. In other words, for liquid droplets of different types of perfume, if the liquid droplets of a plurality of different types of perfume are not controlled to move synchronously, there is no interference between the liquid droplets, and the liquid droplets may move along a same target track or may move along different target tracks. If the movement of the liquid droplets of the plurality of different types of perfume is controlled synchronously, the target tracks of the liquid droplets are different to prevent the movement of the liquid droplets from interfering with each other, that is, the liquid droplets are controlled to move along different target tracks.

In some scenarios, it is necessary to mix liquid droplets of a plurality of different types of perfume to obtain mixed fragrance. In these scenarios, to-be-mixed liquid droplets may be controlled to move to a specified position along their respective target tracks, to complete mixing at the specified position, to obtain a mixed liquid droplet. The fragrance dispersing apparatus may continue to control the mixed liquid droplet to move along a target track to the fragrance dispersing area. For a manner of obtaining the target track of the mixed liquid droplet, refer to the foregoing described manner of obtaining the target track of the liquid droplet. Details are not described herein again. In addition, it should be noted that, in some implementations, the specified position may be on the fragrance dispersing area, that is, the to-be-mixed liquid droplets are controlled to be mixed in the fragrance dispersing area, that is, the mixed liquid droplet is obtained in the fragrance dispersing area.

Subsequently, in some possible application scenarios, to increase interest of the solution, an outer surface of the liquid droplet movement platform may be set to be transparent, so that the user can view a moving process of the liquid droplet. In addition, in some possible implementations, perfume liquid droplets may be liquid droplets with colors, to increase viewing experience of the user.

When the fragrance dispersing apparatus is deployed on an immovable device, the liquid droplet may be controlled to move along the target track based on the digital microfluidic technology. Details are not described herein again. In addition, to ensure that the liquid droplet can move along the target track, a verification mechanism may be added, which is described in detail below.

As described above, the digital microfluidic technology is to apply a voltage to an electrode to drive a liquid droplet to move to a target position at which the electrode is located. It should be noted that, a current that can be detected by the microfluidic circuit and that passes through the electrode when there is a liquid droplet at the target position corresponding to the electrode is different from a current that can be detected by the microfluidic circuit and that passes through the electrode when there is no liquid droplet at the target position corresponding to the electrode. Therefore, this principle can be used to determine a current position at which the liquid droplet is located. For example, in a possible implementation, a current that passes through the electrode when there is no liquid droplet on the electrode is pre-stored in the fragrance dispersing apparatus, or a current that passes through the electrode when there is a liquid droplet on the electrode is pre-stored in the fragrance dispersing apparatus. For example, the current that passes through the electrode when there is a liquid droplet on the electrode is pre-stored in the fragrance dispersing apparatus, a fixed and small voltage is applied to the electrode at a specific position, and a difference between a current that passes through the electrode at this time and the current that passes through the electrode when there is a liquid droplet is compared. If the difference is less than or equal to a threshold, that the liquid droplet is in the position corresponding to the electrode is determined. If the difference is greater than the threshold, that the liquid droplet is not in the position corresponding to the electrode is determined. For another example, in a possible implementation, if the liquid droplet movement platform is large, the liquid droplet is located in a small part of the position, and most of the positions are free of the liquid droplet. Therefore, an abnormal current in currents passing through electrodes in the fragrance dispersing apparatus may be obtained by comparing the currents passing through the electrodes. The abnormal current is a current that is different from most currents. It can be considered that there is a liquid droplet at a position of an electrode corresponding to the abnormal current. If the current position at which the liquid droplet is located is obtained, that is, the position on the target track, the liquid droplet may be controlled to continue to move along the target track. In a possible implementation, the obtained current position at which the liquid droplet is located may be not on the pre-specified target track, and the target track may be replanned. For example, in eight neighborhoods of the position of the liquid droplet, a position closest to the fragrance dispersing area may be used as a target position of the moving droplet, and a voltage is applied to the electrode at the target position to drive the liquid droplet. Then, a position closest to the fragrance dispersing area in eight neighborhoods of the liquid droplet is searched for again as a next target position. A voltage is applied to the electrode at the next target position to drive the liquid droplet to continue to move, and this process is repeated until the liquid droplet moves to the fragrance dispersing area. In a possible implementation, a fixed and small voltage may be applied to the electrode at each position in the specified track, to obtain whether the liquid droplet is in the specified track. If it is determined that there is no liquid droplet at any position in the specified track, the target track may be replanned. In a possible implementation, whether there is a liquid droplet in eight neighborhoods of each position in the specified track may be preferentially verified.

For example, according to a preset order, in the eight neighborhoods of each position in the specified track, whether a liquid droplet is at the position is determined based on a preset direction (for example, clockwise) and by using the same method until the current position at which the liquid droplet is located is found. Alternatively, if all the eight neighborhoods of each position are detected, the process is stopped. If the current position at which the liquid droplet is located has not been determined after the process is stopped, positions other than the eight neighborhoods of each position in the specified track are detected to determine the current position at which the liquid droplet is located.

Finally, to achieve precise control of fragrance dispersing in more scenarios, for example, when the fragrance dispersing apparatus is deployed on a movable device, precise control of fragrance dispersing can be achieved, posture information of the fragrance dispersing apparatus can be obtained, and the liquid droplet is controlled, based on the posture information of the fragrance dispersing apparatus, to move along the target track to the fragrance dispersing area. This is described in detail below.

In this embodiment of this application, the posture information of the fragrance dispersing apparatus may be obtained in a plurality of manners. The posture information is related information used to obtain resistance for the liquid droplet to move along the target track. For example, the posture information may include motion information of the fragrance dispersing apparatus, deflection angle information of the fragrance dispersing apparatus in space, and the like. Specifically, in a preferred implementation, an inertial measurement unit (inertial measurement unit, IMU) may be installed on the fragrance dispersing apparatus or a device on which the fragrance dispersing apparatus is deployed. In this implementation, the posture information may be obtained based on detected IMU data.

In a possible implementation, a motion status of the device may be detected, for example, whether the device flips, vibrates, or shakes is detected. If it is detected that the device is in a moving state, posture information of the device is obtained, and a voltage applied to the electrode in the microfluidic circuit is adjusted based on the posture information of the device, to ensure that the liquid droplet moves normally on the target track. Specifically, when the posture information is obtained based on the IMU data, the IMU data may be received in real time, and a force F of the liquid droplet on the liquid droplet movement platform is estimated based on the IMU data. F is converted into a voltage value V, and a voltage is applied to the electrode at a position opposite to F after adjustment, to prevent the liquid droplet from being thrown out and ensure that the liquid droplet moves on the planned track.

The following describes how to adjust the voltage applied to the electrode based on the posture information, to ensure that the liquid droplet moves along the planned track by using an example.

Assuming that an angle between the device and an x-axis is defined as a, and an angle between the apparatus and a z-axis is defined as β.
(1) A relationship between F and V may be obtained by collecting and fitting experimental data to obtain a mapping relationship V=g(F). During an experiment, a liquid droplet with a fixed size is split on an experimental apparatus, and the relationship between F and V is calculated by adjusting the angle a of the experimental apparatus. Specifically, the voltage applied to the electrode corresponding to the liquid droplet is adjusted to a largest value, and the voltage is gradually reduced, for example, in steps of 5 V, until the liquid droplet slides, and a voltage at this time is recorded as V(a, F). Each piece of a has corresponding F and V, and a parameter of g(x) is estimated through fitting.
(2) A direction of the applied voltage V is determined based on a and β. A correspondence between a or β and the direction of the voltage V may be prestored. For example, Table 1 shows a possible correspondence.

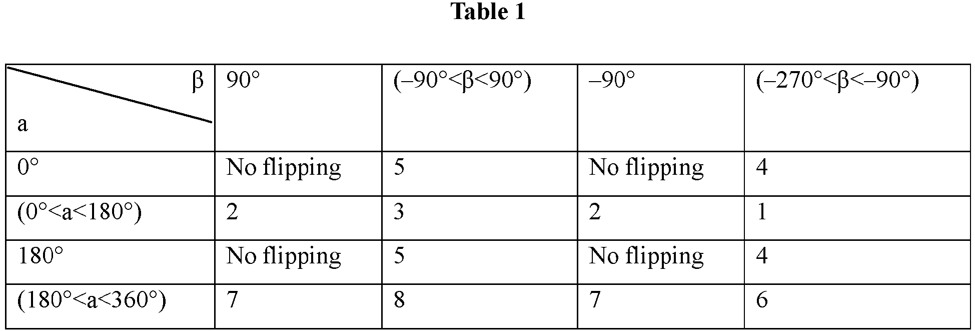

Assuming that positions of eight neighborhoods of the current position of the liquid droplet are respectively a position 1 to a position 8. Specifically, refer to FIG. 7 for understanding. If it is obtained, based on the current posture information of the device, that the current angle a of the device is 0° and the angle β is 90°, it indicates that the device is currently on a horizontal plane and has no roll angle. In this case, there is no need to prevent the liquid droplet from being thrown out, and no additional F needs to be applied. If it is obtained, based on the current posture information of the device, that the current angle a of the device is 0°<a<180° and the angle β is 90°, it indicates that the device has an included angle on the horizontal plane. In this case, the additional F needs to be applied to an electrode corresponding to the position 2, to prevent the liquid droplet from moving from the current position to the position 7. If it is obtained, based on the current posture information of the device, that the current angle a of the device is 180° and the angle β is 90°, it indicates that the device is currently on the horizontal plane. In this case, there is no need to prevent the liquid droplet from being thrown out, and no additional F needs to be applied. If it is obtained, based on the current posture information of the device, that the current angle a of the device is 180°<a<360° and the angle β is 90°, it indicates that the device has an included angle on the horizontal plane and does not have a roll angle. In this case, the additional F needs to be applied to an electrode corresponding to position 7, to prevent the liquid droplet from moving from the current position to the position 2. If it is obtained, based on the current posture information of the device, that the current angle a of the device is 0° and the angle β is -90°<β<90°, it indicates that the device has no included angle on the horizontal plane and has a roll angle. In this case, the additional F needs to be applied to an electrode corresponding to the position 5, to prevent the liquid droplet from moving from the current position to the position 4. If it is obtained, based on the current posture information of the device, that the current angle a of the device is 0°<a<180° and the angle β is -90°<β<90°, it indicates that the device has an included angle on the horizontal plane and has a roll angle. In this case, the additional F needs to be applied to an electrode corresponding to the position 3, to prevent the liquid droplet from moving from the current position to the position 6. If it is obtained, based on the current posture information of the device, that the current angle a of the device is 180° and the angle β is -90°<β<90°, it indicates that the device has an included angle on the horizontal plane and has a roll angle. In this case, the additional F needs to be applied to the electrode corresponding to the position 5, to prevent the liquid droplet from moving from the current position to the position 4. If it is obtained, based on the current posture information of the device, that the current angle a of the device is 180°<a<360° and the angle β is -90°<β<90°, it indicates that the device has an included angle on the horizontal plane and has a roll angle. In this case, the additional F needs to be applied to an electrode corresponding to the position 8, to prevent the liquid droplet from moving from the current position to the position 1. If it is obtained, based on the current posture information of the device, that the current angle a of the device is 0° and the angle β is -90°, it indicates that the device is currently on the horizontal plane. In this case, there is no need to prevent the liquid droplet from being thrown out, and no additional F needs to be applied. If it is obtained, based on the current posture information of the device, that the current angle a of the device is 0°<a<180° and the angle β is -90°, it indicates that the device has an included angle on the horizontal plane. In this case, the additional F needs to be applied to the electrode corresponding to the position 2, to prevent the liquid droplet from moving from the current position to the position 7. If it is obtained, based on the current posture information of the device, that the current angle a of the device is 180° and the angle β is -90°, it indicates that the device is currently on the horizontal plane. In this case, there is no need to prevent the liquid droplet from being thrown out, and no additional F needs to be applied. If it is obtained, based on the current posture information of the device, that the current angle a of the device is 180°<a<360° and the angle β is -90°, it indicates that the device has an included angle on the horizontal plane. In this case, the additional F needs to be applied to the electrode corresponding to the position 7, to prevent the liquid droplet from moving from the current position to the position 2. If it is obtained, based on the current posture information of the device, that the current angle a of the device is 0° and the angle β is -270°<β<-90°, it indicates that the device has no included angle on the horizontal plane and has a roll angle. In this case, the additional F needs to be applied to an electrode corresponding to the position 4, to prevent the liquid droplet from moving from the current position to the position 5. If it is obtained, based on the current posture information of the device, that the current angle a of the device is 0°<a<180° and the angle β is -270°<β<-90°, it indicates that the device has an included angle on the horizontal plane and has a roll angle. In this case, the additional F needs to be applied to an electrode corresponding to the position 1, to prevent the liquid droplet from moving from the current position to the position 8. If it is obtained, based on the current posture information of the device, that the current angle a of the device is 180° and that the angle β is -270°<β<-90°, it indicates that the device has an included angle on the horizontal plane and has a roll angle. In this case, the additional F needs to be applied to the electrode corresponding to the position 4, to prevent the liquid droplet from moving from the current position to the position 5. If it is obtained, based on the current posture information of the device, that the current angle a of the device is 180°<a<360° and the angle β is -270°<β<-90°, it indicates that the device has an included angle on the horizontal plane and has a roll angle. In this case, the additional F needs to be applied to an electrode corresponding to the position 6, to prevent the liquid droplet from moving from the current position to the position 3.

It should be noted that the foregoing Table 1 is merely an optimal correspondence. Actually, there may be another correspondence. In an actual application scenario, the correspondence between a or β and the direction of the voltage V may be set based on an actual requirement.

### 4. Fragrance dispersing area

The fragrance dispersing area is used to speed up volatilization of the liquid droplet.

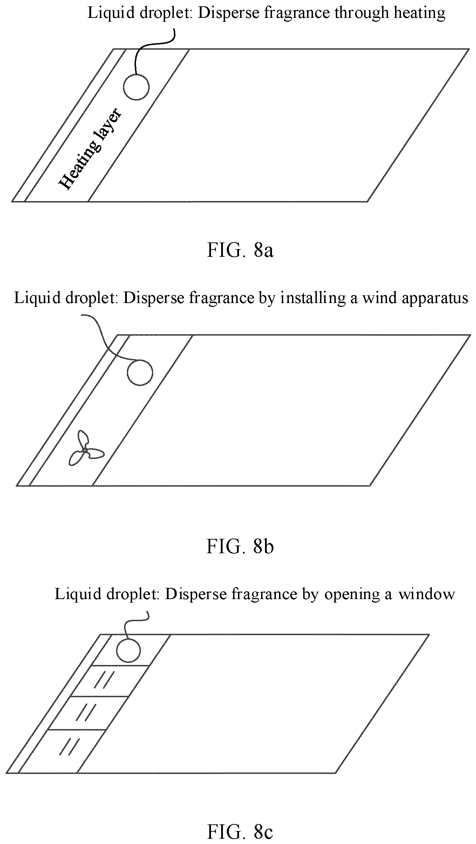

In a possible implementation, the fragrance dispersing area is used to heat the liquid droplet. When the liquid droplet reaches the heating and fragrance dispersing area, the fragrance dispersing area is controlled to heat the liquid droplet, and when there is no liquid droplet in the fragrance dispersing area for a period of time, heating is stopped. In a possible implementation, a small hole is disposed on a shell of the fragrance dispersing area. Refer to FIG. 8a. After the liquid droplet is heated, fragrance corresponding to the liquid droplet disperses from the small hole. It should be noted that, whether there is a liquid droplet in the fragrance dispersing area may be obtained in a manner of current detection. The foregoing has described how to obtain whether there is a liquid droplet in a position corresponding to the electrode. Details are not described herein again.

In a possible implementation, a wind apparatus is disposed in the fragrance dispersing area. When the liquid droplet reaches the heating and fragrance dispersing area, the fragrance dispersing area is controlled to enable the wind apparatus to accelerate volatilization of the liquid droplet, and when there is no liquid droplet in the fragrance dispersing area for a period of time, the wind apparatus is disabled. Refer to FIG. 8b. When the wind apparatus is enabled, that the fragrance corresponding to the liquid droplet disperses from the small hole is accelerated.

In a possible implementation, the liquid droplet may alternatively be naturally volatilized in the fragrance dispersing area. In this implementation, a movable window may be disposed, or a size of the small hole disposed on the shell of the fragrance dispersing area may be increased. When the liquid droplet reaches the heating and fragrance dispersing area, the window is opened. Refer to FIG. 8c. When the window is opened, the fragrance corresponding to the liquid droplet disperses from the window. When there is no liquid droplet in the fragrance dispersing area for a period of time, the window is closed. Alternatively, when the liquid droplet reaches the heating and fragrance dispersing area, the fragrance corresponding to the liquid droplet disperses from the large hole.

It should be noted that, in a possible implementation, fragrance dispersing may be accelerated in the fragrance dispersing area by using a plurality of mechanisms, for example, a manner of heating the liquid droplet and a manner of opening the window may be used at the same time. This is not limited in this embodiment of this application.

The fragrance dispersing apparatus provided in embodiments of this application may interact with the user, and the user may send an instruction to the fragrance dispersing apparatus to indicate when to disperse fragrance, what type of fragrance to disperse, duration for continuously dispersing fragrance, and the like. For example, the following describes an interaction process between the user and the fragrance dispersing apparatus by using several possible application scenarios.

In a possible application scenario, the user may directly send an instruction to the fragrance dispersing apparatus. For example, the user directly operates the fragrance dispersing apparatus, to enable the fragrance dispersing apparatus to obtain the fragrance dispersing instruction. In a possible application scenario, the user may send an instruction to another device, so that the another device sends the instruction to the fragrance dispersing apparatus. For example, the user sends an instruction by operating an APP in a mobile phone, and then a server corresponding to the APP obtains the instruction and sends the instruction to the fragrance dispersing apparatus (for example, a household aromatherapy lamp). The following describes possible application scenarios of embodiments of this application by using a manner in which the user sends the instruction by operating the APP in the mobile phone.

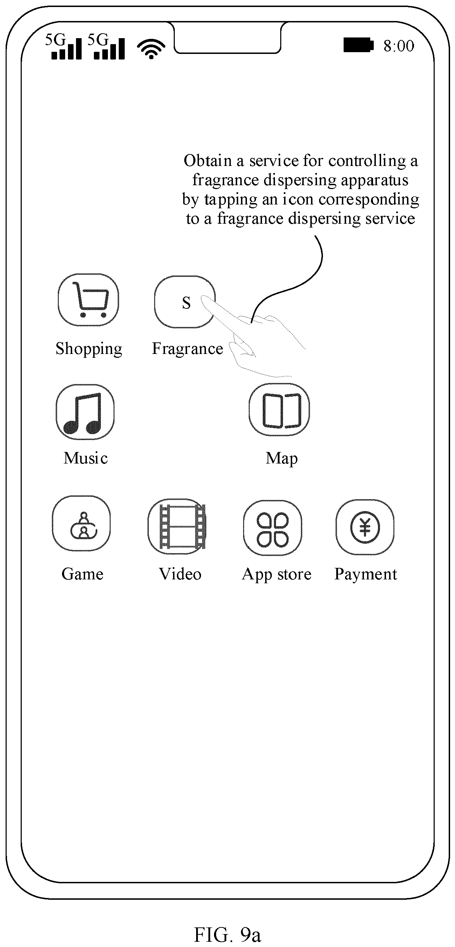

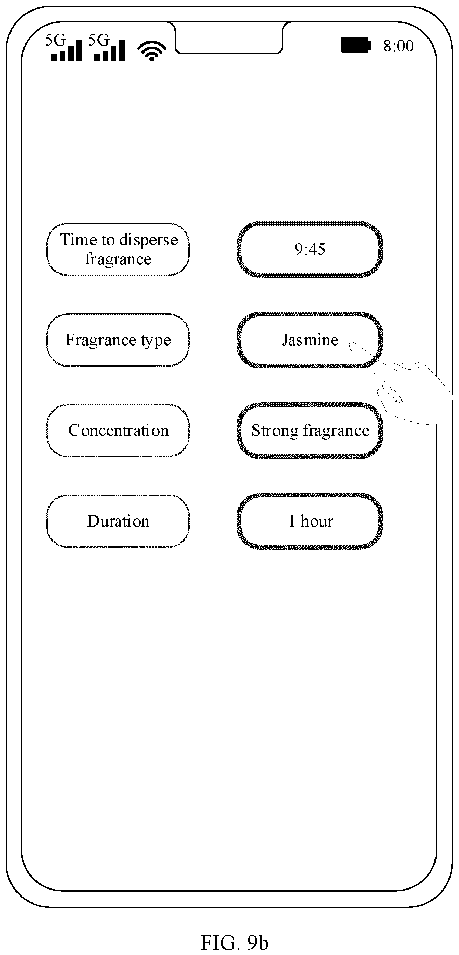

The user may tap the APP to enter an interface for controlling the fragrance dispersing apparatus, for example, enter an interface shown in FIG. 9b from an interface shown in FIG. 9a. The user can freely manage one or more fragrance dispersing apparatuses on the interface. The following uses managing one of the fragrance dispersing apparatuses as an example for description. The user may select time for fragrance dispersing, a fragrance type for fragrance dispersing, concentration of fragrance dispersing, duration of fragrance dispersing, and the like. An example in which the fragrance dispersing instruction indicates to disperse fragrance immediately, the fragrance type is an Atype, and the concentration is strong fragrance is used for description. When the fragrance dispersing apparatus obtains this instruction, candidate liquid is obtained from an area for storing the perfume of type A in the liquid storage tank. Assuming that five liquid droplets are split in the liquid droplet splitting area according to a predetermined strong fragrance standard, and then are mixed in a specified area. A mixed liquid droplet of the five droplets is heated in the heat dissipation area to disperse strong fragrance.

In a possible implementation, the device may further predict, based on information such as an operation instruction sent by the user to the fragrance dispersing apparatus in a historical time period and a historical track of the user, a fragrance dispersing instruction that may be sent by the user at a specific moment, and automatically trigger fragrance dispersing, or predict a current environment of the user, and automatically generate a fragrance dispersing instruction suitable for the environment. For example, when the device obtains that the user attends a friend's party, flower fragrance disperses. When the device obtains that the user is in an office environment, citrus fragrance disperses. When the device detects that the user is exercising (for example, a heart rate of the user is detected by using a wearable device, and that the user is exercising is further determined), refreshing fragrance disperses. For another example, when the device obtains that the user is in a fatigue state (for example, a state of the user is obtained by using a photographing device), soothing and decompressing fragrance disperses.

The fragrance dispersing apparatus provided in embodiments of this application is described above, and a fragrance dispersing control method provided in embodiments of this application is described below.

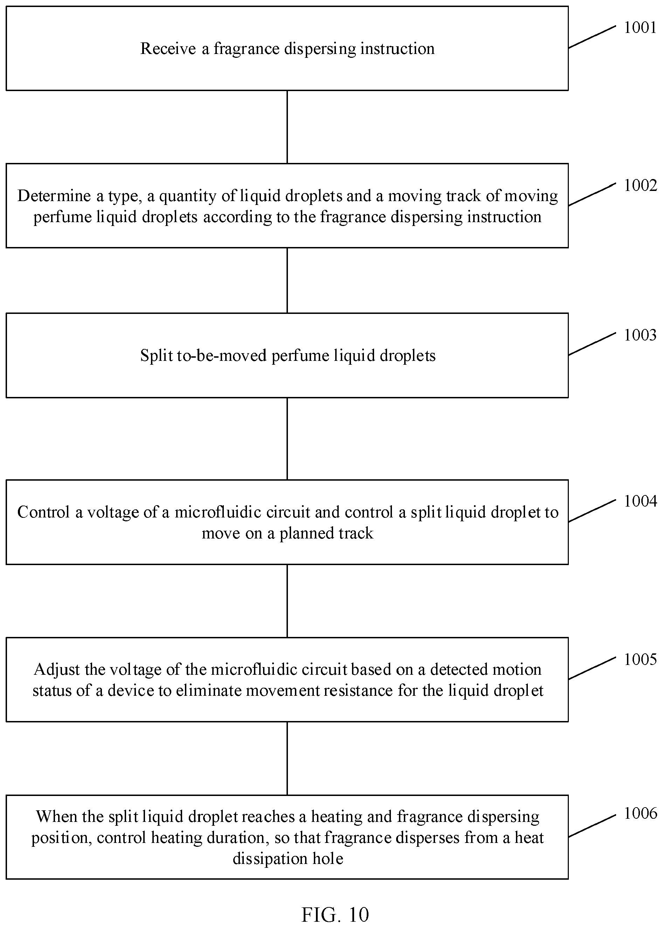

FIG. 10 is a schematic flowchart of a fragrance dispersing control method according to an embodiment of this application.

As shown in FIG. 10, the fragrance dispersing control method according to an embodiment of this application may include the following steps.

1001: Receive a fragrance dispersing instruction.

The fragrance dispersing instruction includes a perfume type (perfume ID identifier may be used), concentration (light fragrance, strong fragrance), and time of fragrance dispersing (for example, a time period of fragrance dispersing, fragrance dispersing duration, or a time interval).

In a possible implementation, an occasion for sending the fragrance dispersing instruction may be triggered by a user by using an operation interface of an APP. Alternatively, the fragrance dispersing apparatus may intelligently sense time to disperse fragrance, a type of fragrance to disperse, and duration for continuously dispersing fragrance. Specifically, for understanding, refer to the foregoing related descriptions that the user may send the instruction or the fragrance dispersing instruction to the fragrance dispersing apparatus. Details are not described herein again.

1002: Determine a type, a quantity of liquid droplets and a moving track of a moving perfume liquid droplet according to the fragrance dispersing instruction.

A quantity of liquid droplets, a liquid type, a planned moving track (target track) of the fragrance dispersing liquid droplet, and a size of a single working droplet (for example, the size of the single droplet is preset to 0.5 µL), and a heating temperature (specifically, the heating temperature may be obtained through calculation based on a liquid droplet volume and fragrance concentration, and in a possible implementation, a range of the heating temperature is [39°C, 100°C]) required for fragrance dispersing this time are determined according to the fragrance dispersing instruction.

In a possible implementation, it is detected whether liquid droplets in a liquid supply area meet a requirement. If not, perfume liquid needed this time is extracted from a storage pool.

1003: Split a to-be-moved perfume liquid droplet.

For understanding, refer to the descriptions of splitting liquid in the liquid supply area to obtain the liquid droplet. This is not repeated herein.

1004: Control a voltage of a microfluidic circuit and control a split liquid droplet to move on a planned track.

The voltage of the microfluidic circuit is controlled to control the split liquid droplet to move on the planned track, for example, move to a heating and fragrance dispersing position close to a fragrance dispersing hole.

1005: Adjust the voltage of the microfluidic circuit based on a detected motion status of a device to eliminate movement resistance for the liquid droplet.

The motion status, for example, flipping, vibration, or shaking, of the device is detected, and the voltage is adjusted to ensure that the working liquid droplet moves normally on the planned track. The foregoing has described in detail how to adjust the voltage applied to the electrode based on the posture information, to ensure that the liquid droplet moves along the planned track. Details are not described herein again.

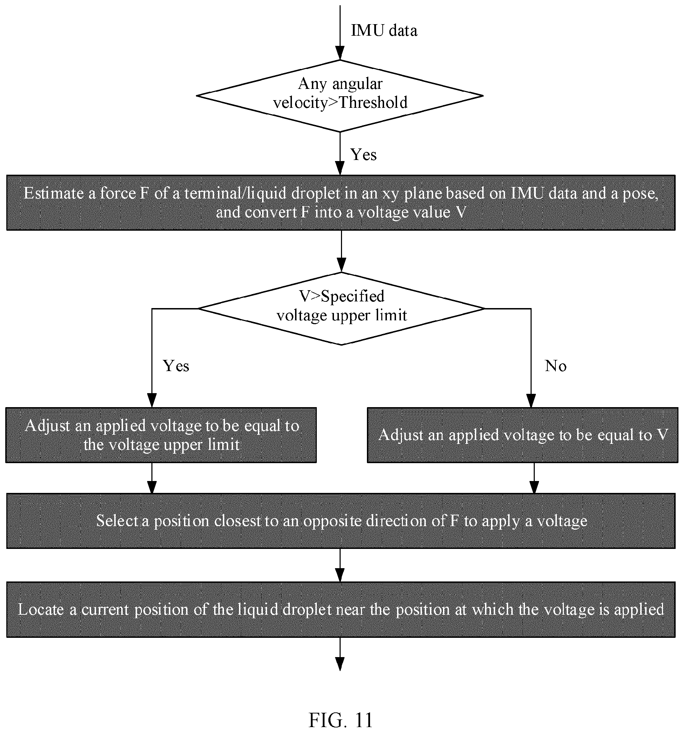

For example, with reference to FIG. 11, the following provides a manner of eliminating movement resistance for the liquid droplet by using IMU data.

Refer to the flowchart shown in FIG. 11. In this implementation, whether any angular velocity of the fragrance dispersing apparatus is greater than a threshold is determined based on the IMU data. If an angular velocity is greater than the threshold, it indicates that the movement resistance for the liquid droplet needs to be eliminated. In this case, a force F of the fragrance dispersing apparatus/liquid droplet on an xy plane is estimated based on the IMU data and the posture information of the device, and F is converted into a voltage value V Specifically, how to obtain V based on F has been described above. Details are not described herein again. If the obtained voltage value V is greater than a specified upper limit value of the voltage value, the voltage applied to the corresponding electrode is the upper limit value of the voltage value. If the obtained voltage value V is not greater than the specified upper limit value of the voltage value, the voltage applied to the corresponding electrode is the obtained voltage value V An electrode corresponding to a position closest to an opposite direction of F is selected to apply a voltage. Specifically, how to obtain a direction of a force is described above. For details, refer to Table 1. Details are not described herein again. In addition, a verification mechanism may be further added to locate a current position of the liquid droplet near the position where the voltage is applied. Specifically, the verification mechanism has been described above. Details are not described herein again.

1006: When the split liquid droplet reaches the heating and fragrance dispersing position, control heating duration, so that fragrance disperses from the heat dissipation hole.

When the liquid droplet reaches the heating and fragrance dispersing position, a heating area is controlled to heat the liquid droplet, and when there is no working liquid droplet in the heating area for a period of time, heating is stopped. When the liquid droplet is heated, corresponding fragrance disperses from the heat dissipation hole.

In this embodiment of this application, a plurality of perfume types are further supported, and a fragrance dispersing manner of perfume mixing is supported. This has been described above, and a specific implementation is provided herein.

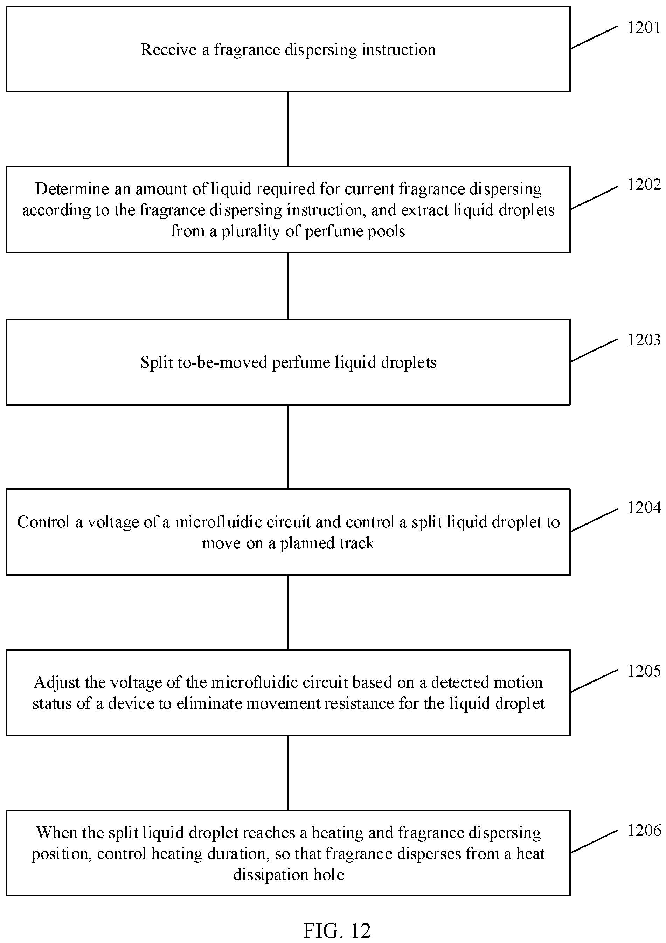

FIG. 12 is a schematic flowchart of a fragrance dispersing control method according to an embodiment of this application.

As shown in FIG. 12, a fragrance dispersing control method according to an embodiment of this application includes the following steps.

1201: Receive a fragrance dispersing instruction.

When the fragrance dispersing instruction is received, fragrance dispersing is triggered, and liquid may be extracted from different perfume pools according to requirements. There may be a plurality of perfume IDs in the fragrance dispersing instruction, to be specific, this indicates that fragrance of the current perfume needs to be mixed. Optionally, a plurality of perfume IDs to be extracted this time may also be determined based on a mapping table between a perfume ID and a mixed perfume corresponding to a perfume ID.

1202: Determine an amount of liquid required for fragrance dispersing according to the fragrance dispersing instruction, and extract liquid droplets from a plurality of perfume pools.

In a possible implementation, it is detected whether liquid droplets in a liquid supply area meet a requirement. If not, perfume liquid needed this time is extracted from a storage pool.

1203: Split a to-be-moved perfume liquid droplet.

1204: Control a voltage of a microfluidic circuit and control a split liquid droplet to move on a planned track.

1205: Adjust the voltage of the microfluidic circuit based on a detected motion status of a device to eliminate movement resistance for the liquid droplet.

1206: When the split liquid droplet reaches a heating and fragrance dispersing position, control heating duration, so that fragrance disperses from a heat dissipation hole.

Step 1203 to step 1206 may be understood with reference to step 1003 to step 1006 in the embodiment corresponding to FIG. 10. Details are not described herein again.

In a possible implementation, before liquid splitting is performed in step 1202, liquid droplets from a plurality of perfume pools are mixed into one liquid droplet, and then splitting is performed.

In a possible implementation, after the liquid droplets are extracted from the plurality of perfume pools in step 1202, liquid droplet splitting may also be performed separately in step 1203. Then, through steps 1204 to 1206, the plurality of liquid droplets are controlled to move to a heating area, and before heating, the plurality of split liquid droplets are mixed and finally heated.

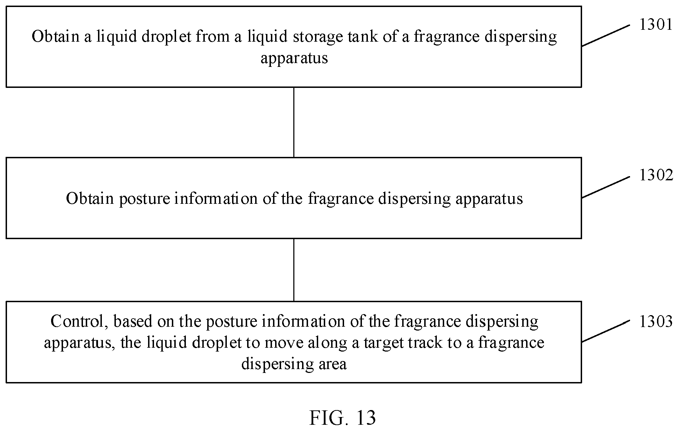

FIG. 13 is a schematic flowchart of a fragrance dispersing control method according to an embodiment of this application.

As shown in FIG. 13, the fragrance dispersing control method according to an embodiment of this application includes the following steps.

1301: Obtain a liquid droplet from a liquid storage tank of a fragrance dispersing apparatus.

The liquid storage tank and how to obtain a liquid droplet from the liquid storage tank have been described above. Details are not described herein again.

1302: Obtain posture information of the fragrance dispersing apparatus.

A definition of the posture information and how to obtain the posture information of the fragrance dispersing apparatus have been described above. Details are not described herein again.

1303: Control, based on the posture information of the fragrance dispersing apparatus, the liquid droplet to move along a target track to a fragrance dispersing area.

How to control, based on the posture information of the fragrance dispersing apparatus, the liquid droplet to move along the target track to the fragrance dispersing area has been described above. Details are not described herein again.

In a possible implementation, the liquid droplet is heated in the fragrance dispersing area.

In a possible implementation, controlling, based on the posture information of the fragrance dispersing apparatus, the liquid droplet to move along a target track to a heating area includes:
obtaining, based on the current posture information of the fragrance dispersing apparatus, resistance for the liquid droplet to move along the target track.

The liquid droplet is driven, based on the resistance and a first driving force, to move along the target track. The first driving force is a driving force that drives the liquid droplet to move along the target track when the fragrance dispersing apparatus is stationary on a horizontal plane.

In a possible implementation, obtaining a liquid droplet from a liquid storage tank of a fragrance dispersing apparatus includes:
obtaining the liquid droplet from the liquid storage tank of the fragrance dispersing apparatus based on a first extraction apparatus. The first extraction apparatus includes at least one of a capillary tube, a liquid pump or a combination apparatus. The combination apparatus includes a valve and a microfluidic circuit. One end of the valve is connected to the liquid storage tank, and the other end of the valve is connected to the microfluidic circuit. When the valve is open, the liquid droplet is obtained from the liquid storage tank by using the microfluidic circuit.

In a possible implementation, obtaining a liquid droplet from a liquid storage tank of a fragrance dispersing apparatus includes:
obtaining a plurality of liquid droplets from different liquid storage tanks of the fragrance dispersing apparatus.

Controlling, based on the posture information of the fragrance dispersing apparatus, the liquid droplet to move along a target track to a heating area includes:
controlling, based on the posture information of the fragrance dispersing apparatus, at least two of the plurality of liquid droplets to move along respective target tracks to a specific position, to obtain a mixed liquid droplet.

The mixed liquid droplet is controlled, based on the posture information of the fragrance dispersing apparatus, to move along a target track of the mixed liquid droplet to the heating area.

In a possible implementation, obtaining a liquid droplet from a liquid storage tank of a fragrance dispersing apparatus includes:
obtaining respectively a single droplet or a plurality of liquid droplets from each of at least two liquid storage tanks of the fragrance dispersing apparatus.

Controlling, based on the posture information of the fragrance dispersing apparatus, the liquid droplet to move along a target track to a heating area includes:
controlling, based on the posture information of the fragrance dispersing apparatus, at least two of the plurality of liquid droplets to move along respective target tracks to a specific position in the heating area, to obtain a mixed liquid droplet.

Heating a liquid droplet in the heating area includes:
heating the mixed liquid droplet in the heating area.

In a possible implementation, the method further includes:
obtaining a first instruction, where the first instruction indicates at least one of a fragrance type, fragrance concentration, or fragrance dispersing duration.

Obtaining a liquid droplet from a liquid storage tank of a fragrance dispersing apparatus includes:
obtaining at least one liquid droplet from at least one liquid storage tank of the fragrance dispersing apparatus in response to the first instruction.

In a possible implementation, the method further includes:
obtaining a second instruction, where the second instruction indicates at least one target track.

Controlling, based on the posture information of the fragrance dispersing apparatus, the liquid droplet to move along a target track to a heating area includes:
controlling, based on the posture information of the fragrance dispersing apparatus in response to the second instruction, at least one liquid droplet to move along at least one target track to the heating area.

In a possible implementation, the obtaining a liquid droplet from a liquid storage tank of a fragrance dispersing apparatus includes:
obtaining, the liquid droplet from the liquid storage tank of the fragrance dispersing apparatus by using the microfluidic circuit.

In a possible implementation, the fragrance dispersing apparatus is deployed on at least one of a mobile phone case, a mobile phone rear cover, a subscriber identity module SIM card tray, an in-vehicle apparatus, or a robot.

In a possible implementation, an inertial measurement unit IMU is deployed on the fragrance dispersing apparatus, and obtaining the posture information of the fragrance dispersing apparatus includes:
obtaining the posture information of the fragrance dispersing apparatus based on the IMU.

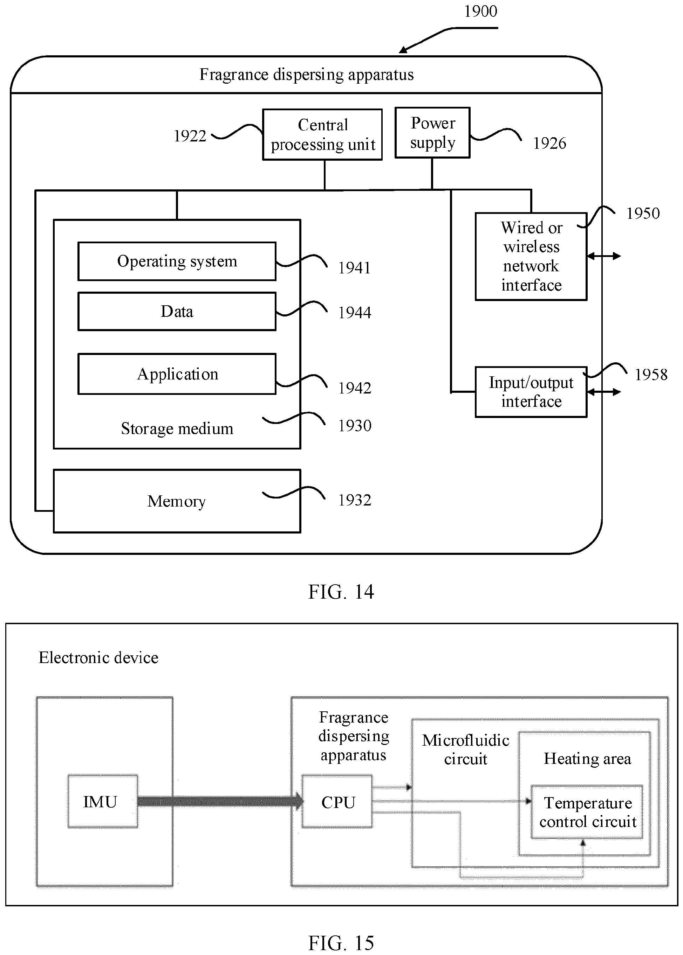

FIG. 14 is a schematic diagram of a structure of another fragrance dispersing apparatus according to this application.

An embodiment of this application further provides a fragrance dispersing apparatus. FIG. 14 is a schematic diagram of a structure of a fragrance dispersing apparatus according to an embodiment of this application. The fragrance dispersing apparatus described in embodiments corresponding to FIG. 2a to FIG. 8c may be deployed on the fragrance dispersing apparatus 1900, and the fragrance dispersing apparatus 1900 is configured to implement a function of the fragrance dispersing apparatus in embodiments corresponding to FIG. 2a to FIG. 8c. Specifically, the fragrance dispersing apparatus 1900 may vary greatly due to different configurations or performance, and may include one or more central processing units CPUs 1922 (for example, one or more processors) and a memory 1932, and one or more storage media 1930 (for example, one or more mass storage devices) for storing an application 1942 or data 1944.

The memory 1932 and the storage media 1930 may be used for temporary storage or persistent storage. In an embodiment, the memory 1932 is a random access memory RAM, and may directly exchange data with the central processing unit 1922, is configured to load the data 1944 and the application 1942 and/or an operating system 1941 for the central processing unit 1922 to directly run and use, and is usually used as a temporary data storage medium of the operating system or another running program. A program stored in the storage media 1930 may include one or more modules (not shown in FIG. 14), and each module may include a series of instruction operations on the fragrance dispersing apparatus.

Further, the central processing unit 1922 may be configured to communicate with the storage media 1930, and perform, on the fragrance dispersing apparatus 1900, the series of instruction operations in the storage media 1930. In a possible implementation, the storage media 1930 store program instructions and data corresponding to the method steps shown in any one of embodiments in FIG. 6 or FIG. 7.

The fragrance dispersing apparatus 1900 may further include one or more power supplies 1926, one or more wired or wireless network interfaces 1950, one or more input/output interfaces 1958, and/or one or more operating systems 1941, for example, Windows ServerTM, Mac OS XTM, UnixTM, LinuxTM, and FreeBSDTM.

In a possible implementation, the central processing unit 1922 is configured to perform steps performed by the fragrance dispersing apparatus shown in any one of the foregoing embodiments in FIG. 2c to FIG. 13. There may be one or more central processing units.

It should be understood that, the foregoing is merely an example provided in this embodiment of this application. Moreover, the fragrance dispersing apparatus may have more or fewer components than those shown, may combine two or more components, or may have different configurations of components. For example, FIG. 15 is a system diagram of installing a fragrance dispersing apparatus on a specific terminal device, for example, the fragrance dispersing apparatus is stalled on a mobile phone (for example, a rear cover of the mobile phone or a SIM card). Alternatively, FIG. 15 is a system diagram of communication between a mobile phone and an independent module. When the fragrance dispersing apparatus is installed on a specific terminal device, the terminal device may be regarded as a large fragrance dispersing apparatus. A microprocessor for receiving IMU data is disposed in the fragrance dispersing apparatus, to control, by using a microfluidic circuit, a micro droplet to move on a predetermined plan track; and the heating area is controlled to heat the liquid droplet. The microfluidic circuit is configured to receive instructions from a CPU, to perform voltage control, detect a position of a liquid droplet, provide the position to the CPU, control a switch of an electrode circuit, and achieve movement of the liquid droplet. The IMU is used to detect and collect pose data of movement of the device, and send the data to the CPU to assist in liquid droplet movement control.

All or some of the foregoing embodiments may be implemented by using software, hardware, firmware, or any combination thereof. When the software is used to implement embodiments, all or some of embodiments may be implemented in a form of a computer program product.

A training apparatus/the fragrance dispersing apparatus in embodiments of this application may be a chip. The chip includes a processing unit and a communication unit. The processing unit may be, for example, a processor, and the communication unit may be, for example, an input/output interface, a pin, or a circuit. When the training apparatus is the chip, the processing unit may execute computer-executable instructions stored in a storage unit, so that the chip performs the methods described in embodiments shown in FIG. 3 and FIG. 5. In another possible implementation, when the fragrance dispersing apparatus is the chip, the chip performs the methods described in embodiments shown in FIG. 6 and FIG. 7. Optionally, the storage unit is a storage unit in the chip, for example, a register or a cache. Alternatively, the storage unit may be a storage unit located outside the chip and in a wireless access device end, for example, a read-only memory (read-only memory, ROM) or another type of static storage device that can store static information and instructions, or a random access memory (random access memory, RAM).

Specifically, the processing unit or the processor may be a central processing unit (central processing unit, CPU), a neural-network processing unit (neural-network processing unit, NPU), a graphics processing unit (graphics processing unit, GPU), a digital signal processor (digital signal processor, DSP), an application-specific integrated circuit (application-specific integrated circuit, ASIC), a field programmable gate array (field programmable gate array, FPGA), another programmable logic device, a discrete gate, a transistor logic device, a discrete hardware component, or the like. A general purpose processor may be a microprocessor or any regular processor or the like.

In addition, it should be noted that the described apparatus embodiment is merely an example. The units described as separate parts may or may not be physically separate, and parts displayed as units may or may not be physical units, may be located in one position, or may be distributed on a plurality of network units. Some or all the modules may be selected based on an actual requirement to achieve objectives of solutions of embodiments. In addition, in accompanying drawings of the apparatus embodiments provided by this application, connection relationships between modules indicate that the modules have communication connections with each other, which may be specifically implemented as one or more communication buses or signal cables.

Based on the descriptions of the foregoing implementations, a person skilled in the art may clearly understand that this application may be implemented by software in addition to necessary universal hardware, or by dedicated hardware, including a dedicated integrated circuit, a dedicated CPU, a dedicated memory, a dedicated component, and the like. Generally, any functions that can be performed by a computer program can be easily implemented by using corresponding hardware. Moreover, a specific hardware structure used to achieve a same function may be in various forms, for example, in a form of an analog circuit, a digital circuit, or a dedicated circuit. However, as for this application, software program implementation is a preferred implementation in most cases. Based on such an understanding, the technical solutions of this application essentially or the part contributing to the conventional technology may be implemented in a form of a software product. The computer software product is stored in a readable storage medium, for example, a floppy disk, a USB flash drive, a removable hard disk, a read-only memory (read-only memory, ROM), a random access memory (random access memory, RAM), a magnetic disk, or an optical disc of a computer, and includes several instructions for instructing a computer device (which may be a personal computer, a server, a network device, or the like) to perform the methods described in embodiments of this application.

All or some of the foregoing embodiments may be implemented by using software, hardware, firmware, or any combination thereof. When the software is used to implement embodiments, all or some of embodiments may be implemented in a form of a computer program product.

An embodiment of this application further provides a computer-readable storage medium. The computer-readable storage medium stores a program for training a model. When the program is run on a computer, the computer is enabled to perform the steps in the methods described in embodiments shown in FIG. 3 and FIG. 5.

An embodiment of this application further provides a computer-readable storage medium. The computer-readable storage medium stores a program for data processing. When the program is run on a computer, the computer is enabled to perform the steps in the methods described in embodiments shown in FIG. 6 and FIG. 7.

An embodiment of this application further provides a digital processing chip. A circuit and one or more interfaces that are configured to implement the processor or a function of the processor are integrated into the digital processing chip. When a memory is integrated into the digital processing chip, the digital processing chip may implement the method steps in any one or more embodiments in the foregoing embodiments. When a memory is not integrated into the digital processing chip, the digital processing chip may be connected to an external memory through a communication interface. The digital processing chip implements, based on program code stored in the external memory, an action performed by the training apparatus/fragrance dispersing apparatus in the foregoing embodiment.

An embodiment of this application further provides a computer program product. The computer program product includes one or more computer instructions. When the computer program instructions are loaded and executed on a computer, the procedures or functions according to embodiments of this application are all or partially generated. The computer may be a general-purpose computer, a special-purpose computer, a computer network, or another programmable apparatus. The computer instructions may be stored in a computer-readable storage medium or may be transmitted from a computer-readable storage medium to another computer-readable storage medium. For example, the computer instructions may be transmitted from a website, computer, server, or data center to another website, computer, server, or data center in a wired (for example, a coaxial cable, an optical fiber, or a digital subscriber line (DSL)) or wireless (for example, infrared, radio, or microwave) manner. The computer-readable storage medium may be any usable medium accessible by a computer, or a data storage device, such as a server or a data center, integrating one or more usable media. The usable medium may be a magnetic medium (for example, a floppy disk, a hard disk, or a magnetic tape), an optical medium (for example, a DVD), a semiconductor medium (for example, a solid state disk Solid State Disk (SSD)), or the like.

A person of ordinary skill in the art may understand that all or some of the steps of the methods in embodiments may be implemented by a program instructing relevant hardware. The program may be stored in a computer-readable storage medium. The storage medium may include: a ROM, a RAM, a magnetic disk, or an optical disc.

The foregoing describes in detail the method for identifying a root cause faulty node and a related apparatus provided in embodiments of this application. Specific examples are used in this specification to describe principles and implementations of this application. Descriptions in the foregoing embodiments are merely used to help understand the method and a core idea of this application. In addition, a person of ordinary skill in the art may make modifications to specific implementations and the application scope according to the idea of this application. In conclusion, the content of this specification shall not be construed as a limitation on this application.

In the specification, claims, and the accompanying drawings of this application, the terms "first", "second", and the like are intended to distinguish between similar objects but do not necessarily indicate a specific order or sequence. It should be understood that data used in such a way may be interchangeable in proper circumstances, so that embodiments of the present invention described herein can be implemented in other orders than the order illustrated or described herein. The term "and/or" in this application describes only an association relationship between associated objects and represents that three relationships may exist. For example, A and/or B may represent the following three cases: Only A exists, both A and B exist, and only B exists. In addition, the character "/" in this specification generally indicates an "or" relationship between the associated objects. In addition, terms "include", "have" and any other variants mean to cover non-exclusive inclusion, for example, a process, method, system, product, or device that includes a series of steps or modules is not necessarily limited to those steps or modules, but may include other steps or modules not explicitly listed or inherent to such a process, method, product, or device. Names or numbers of steps in this application do not mean that the steps in the method procedure need to be performed in a time/logical sequence indicated by the names or numbers. An execution sequence of the steps in the procedure that have been named or numbered can be changed based on a technical objective to be achieved, provided that same or similar technical effect can be achieved. Division into the modules in this application is logical division. In actual application, there may be another division manner. For example, a plurality of modules may be combined or integrated into another system, or some features may be ignored or may not be performed. In addition, the displayed or discussed mutual coupling or direct coupling or communication connection may be through some ports, and the indirect coupling or communication connection between modules may be in an electrical form or another similar form. This is not limited in this application. In addition, modules or sub-modules described as separate components may be or may not be physically separated, or may be or may not be physical modules, or may not be grouped into a plurality of circuit modules. Objectives of the solutions of this application may be achieved by selecting some or all of the modules according to actual requirements.

In the description of this application, "a plurality of" means two or more than two, unless otherwise specifically limited.

In this application, unless otherwise expressly specified and limited, terms such as "installed", "connected to", "connected", "fixed", and "set" should be understood in a broad sense, for example, may be a fixed connection, may be a detachable connection, or may be integration; may be a mechanical connection, or may be an electrical connection; and may be a direct connection, may be an indirect connection through an intermediate medium, or may be internal communication between two elements or an interaction relationship between two elements. A person of ordinary skill in the art may interpret specific meanings of the foregoing terms in this application according to specific cases.

In the descriptions of this application, it should be understood that an orientation or a position relationship indicated by a term, for example, "length", "width", "above", "below", "front", "behind", "left", "right", "vertical", "horizontal", "top", "bottom", "inside", or "outside" is an orientation or a position relationship shown based on the accompanying drawings, and is merely intended to describe this application and simplify the description, but is not intended to indicate or imply that an indicated apparatus or component shall have a specific orientation or be formed and operated in a specific orientation, and therefore cannot be understood as a limitation on this application.

Some terms are used in the specification and the claims to refer to particular components. A person skilled in the art should be able to understand that hardware manufacturers may name a same component by using different terms. In the specification and the subsequent claims, components are not differentiated based on a difference between names; instead, a difference between functions of the components is used as a differentiation criterion. The "including" or "comprising" mentioned in the specification and the claims is an open-ended term, and therefore shall be construed as "including but not limited to" or "comprising but not limited to".

## Claims

1. A fragrance dispersing control method, wherein the fragrance dispersing control method is applied to a fragrance dispersing apparatus, and comprises:
obtaining at least one liquid droplet from a liquid storage tank of the fragrance dispersing apparatus;
obtaining posture information of the fragrance dispersing apparatus; and
controlling, based on the posture information of the fragrance dispersing apparatus, the at least one liquid droplet to move along a respective target track to a fragrance dispersing area.

2. The method according to claim 1, wherein the method further comprises:
heating the at least one liquid droplet in the fragrance dispersing area.

3. The method according to claim 1 or 2, wherein the controlling, based on the posture information of the fragrance dispersing apparatus, the at least one liquid droplet to move along a respective target track to a fragrance dispersing area comprises:
controlling, based on the posture information of the fragrance dispersing apparatus by using a first microfluidic circuit, the at least one liquid droplet to move along the respective target track to the fragrance dispersing area.

4. The method according to claim 3, wherein the controlling, based on the posture information of the fragrance dispersing apparatus by using a first microfluidic circuit, the at least one liquid droplet to move along the respective target track to the fragrance dispersing area comprises:
obtaining, based on the current posture information of the fragrance dispersing apparatus, resistance for the at least one liquid droplet to move along the respective target track; and
driving, based on the resistance and a first driving force by using the first microfluidic circuit, the at least one liquid droplet to move along the respective target track to the fragrance dispersing area, wherein the first driving force comprises a driving force that drives the at least one liquid droplet to move along the respective target track on a horizontal plane.

5. The method according to claim 4, wherein the first driving force indicates to apply a first voltage value at a first target position, and the driving, based on the resistance and a first driving force by using the first microfluidic circuit, the at least one liquid droplet to move along the respective target track to the fragrance dispersing area comprises:
obtaining a second driving force based on the resistance and a mapping relationship, wherein the second driving force indicates to apply a second voltage value at a second target position, and the mapping relationship comprises a mapping between the resistance, the second target position, and the second voltage value; and
simultaneously applying the first voltage value at the first target position and applying the second voltage value at the second target position by using the first microfluidic circuit, to drive the at least one liquid droplet to move along the respective target track to the fragrance dispersing area.

6. The method according to any one of claims 1 to 5, wherein the obtaining at least one liquid droplet from a liquid storage tank of the fragrance dispersing apparatus comprises:
obtaining the liquid droplet from the liquid storage tank of the fragrance dispersing apparatus by using a capillary tube or a liquid pump.

7. The method according to any one of claims 1 to 5, wherein the obtaining at least one liquid droplet from a liquid storage tank of the fragrance dispersing apparatus comprises:
obtaining the at least one liquid droplet from the liquid storage tank of the fragrance dispersing apparatus by using a second microfluidic circuit.

8. The method according to claim 7, wherein the obtaining the liquid droplet from the liquid storage tank of the fragrance dispersing apparatus by using a second microfluidic circuit comprises:
obtaining candidate liquid from the liquid storage tank of the fragrance dispersing apparatus; and
splitting the candidate liquid by using the second microfluidic circuit to obtain the liquid droplet.

9. The method according to any one of claims 1 to 8, wherein the obtaining at least one liquid droplet from a liquid storage tank of the fragrance dispersing apparatus comprises:
obtaining a plurality of liquid droplets from different liquid storage tanks of the fragrance dispersing apparatus; and
the controlling, based on the posture information of the fragrance dispersing apparatus, the at least one liquid droplet to move along a respective target track to a fragrance dispersing area comprises:
controlling, based on the posture information of the fragrance dispersing apparatus, at least two of the plurality of liquid droplets to move along respective target tracks to a same position to obtain a mixed liquid droplet; and
controlling, based on the posture information of the fragrance dispersing apparatus, the mixed liquid droplet to move along a target track of the mixed liquid droplet to the fragrance dispersing area.

10. The method according to any one of claims 1 to 8, wherein the obtaining at least one liquid droplet from a liquid storage tank of the fragrance dispersing apparatus comprises:
obtaining a plurality of liquid droplets from different liquid storage tanks of the fragrance dispersing apparatus; and
the controlling, based on the posture information of the fragrance dispersing apparatus, the at least one liquid droplet to move along a respective target track to a fragrance dispersing area comprises:
controlling, based on the posture information of the fragrance dispersing apparatus, at least two of the plurality of liquid droplets to move along respective target tracks to a same position in the fragrance dispersing area.

11. The method according to any one of claims 1 to 10, wherein the method further comprises:
obtaining a first instruction, wherein the first instruction indicates at least one of a fragrance type, fragrance concentration, or fragrance dispersing duration; and
the obtaining at least one liquid droplet from a liquid storage tank of the fragrance dispersing apparatus comprises:
obtaining the at least one liquid droplet from at least one liquid storage tank of the fragrance dispersing apparatus in response to the first instruction.

12. The method according to any one of claims 1 to 11, wherein the method further comprises:
obtaining a second instruction, wherein the second instruction indicates at least one target track; and
the controlling, based on the posture information of the fragrance dispersing apparatus, the at least one liquid droplet to move along a respective target track to a fragrance dispersing area comprises:
controlling, based on the posture information of the fragrance dispersing apparatus in response to the second instruction, the at least one liquid droplet to move along the at least one target track to the fragrance dispersing area.

13. The method according to any one of claims 1 to 12, wherein an inertial measurement unit IMU is deployed on the fragrance dispersing apparatus, and the obtaining posture information of the fragrance dispersing apparatus comprises:
obtaining the posture information of the fragrance dispersing apparatus based on IMU data.

14. A fragrance dispersing apparatus, comprising a liquid storage tank, a processor, a liquid droplet splitting apparatus, and a liquid droplet movement platform, wherein
the liquid droplet splitting apparatus is configured to obtain at least one liquid droplet from the liquid storage tank of the fragrance dispersing apparatus; and
the processor is configured to control, based on posture information of the fragrance dispersing apparatus, the at least one liquid droplet to move from the liquid droplet movement platform to a fragrance dispersing area along a target track.

15. The apparatus according to claim 14, wherein the fragrance dispersing apparatus further comprises a heating apparatus, and the heating apparatus is configured to:
heat the at least one liquid droplet in the fragrance dispersing area.

16. The apparatus according to claim 14 or 15, wherein the processor is specifically configured to:
control, based on the posture information of the fragrance dispersing apparatus by using a first microfluidic circuit, the at least one liquid droplet to move to the fragrance dispersing area along a respective target track.

17. The apparatus according to claim 16, wherein the processor is specifically configured to:
obtain, based on the current posture information of the fragrance dispersing apparatus, resistance for the at least one liquid droplet to move along the respective target track; and
drive, based on the resistance and a first driving force by using the first microfluidic circuit, the at least one liquid droplet to move along the respective target track to the fragrance dispersing area, wherein the first driving force comprises a driving force that drives the at least one liquid droplet to move along the respective target track on a horizontal plane.

18. The apparatus according to any one of claims 14 to 17, wherein the liquid droplet splitting apparatus is specifically configured to:
obtain a plurality of liquid droplets from different liquid storage tanks of the fragrance dispersing apparatus; and
the processor is specifically configured to:
control, based on the posture information of the fragrance dispersing apparatus, at least two of the plurality of liquid droplets to move along respective target tracks to a same position to obtain a mixed liquid droplet; and
control, based on the posture information of the fragrance dispersing apparatus, the mixed liquid droplet to move along a target track of the mixed liquid droplet to the fragrance dispersing area.

19. The apparatus according to any one of claims 14 to 17, wherein the liquid droplet splitting apparatus is specifically configured to:
obtain a plurality of liquid droplets from different liquid storage tanks of the fragrance dispersing apparatus; and
the processor is specifically configured to:
control, based on the posture information of the fragrance dispersing apparatus, at least two of the plurality of liquid droplets to move along respective target tracks to a same position in the fragrance dispersing area.

20. The apparatus according to any one of claims 14 to 19, wherein the liquid droplet splitting apparatus is specifically configured to:
obtain at least one liquid droplet from at least one liquid storage tank of the fragrance dispersing apparatus in response to a first instruction, wherein the first instruction indicates at least one of a fragrance type, fragrance concentration, or fragrance dispersing duration.

21. The apparatus according to any one of claims 14 to 20, wherein the processor is specifically configured to:
control, based on the posture information of the fragrance dispersing apparatus in response to a second instruction, at least one liquid droplet to move along at least one target track to the fragrance dispersing area, wherein the second instruction indicates the at least one target track.

22. The apparatus according to any one of claims 14 to 21, wherein the apparatus further comprises a housing, the liquid droplet movement platform is accommodated in the housing, and transparency of the housing is greater than a threshold.

23. The apparatus according to any one of claims 14 to 22, wherein the fragrance dispersing apparatus is deployed on at least one of a mobile phone case, a mobile phone rear cover, a subscriber identity module SIM card tray, an electronic device, an in-vehicle apparatus, or a robot.

24. A fragrance dispersing apparatus, comprising:
a memory, configured to store computer-readable instructions; and
a processor coupled to the memory, configured to execute the computer-readable instructions in the memory, to perform the method according to any one of claims 1 to 13.

25. A computer-readable storage medium, comprising instructions, wherein when the instructions are run on a computer, the computer is enabled to perform the method according to any one of claims 1 to 13.

26. A computer program product, wherein when the computer program product runs on one or more processors, the method according to any one of claims 1 to 13 is performed.

27. A chip, wherein the chip is coupled to a memory, and is configured to execute a program stored in the memory, to perform the method according to any one of claims 1 to 13.
